# EUROPEAN PATENT APPLICATION

(11) **EP 4 523 625 A1**
(43) Date of publication of application: **19.03.2025**
(21) Application number: 23803373.2
(22) Date of filing: 20.04.2023
(51) Int. Cl.: A61B 5/257, A61B 5/262, A61B 5/266, A61B 5/273, A61N 1/04

(54) **ELECTRODE DEVICE FOR LIVING BODY**

(30) Priority: 10.05.2022 JP 2022077435; 14.12.2022 JP 2022199715
(71) Applicant: Toppan Holdings Inc., Tokyo 110-0016 (JP)
(72) Inventor: YAMAGUCHI, Hirotaka, Tokyo 110-0016 (JP); UEDA, Ryuji, Tokyo 110-0016 (JP); KINA, Osamu, Tokyo 110-0016 (JP); UEMURA, Daizo, Tokyo 110-0016 (JP)
(74) Representative: TBK
(86) International application number: PCT/JP2023/015730
(87) International publication number: WO 2023/218891

(57) **Abstract**

An object of the present invention is to provide a structure of an electrode sheet having a low resistance, and to strengthen fixation between the skin, an electrode, and a sensor module and thus reduce noise attributed to moments of force applied, and to reduce wearing sensation felt by a subject having the electrode sheet attached thereto. Therefore, an electrode device, according to the present invention, for a living body includes: an electrode sheet including at least a pair of electrodes that receives a bioelectrical signal, a wiring member that transmits the bioelectrical signal received, a sensor module that outputs a signal relating to the bioelectrical signal to an exterior, a connector that connects the bioelectrical signal transmitted, to the sensor module, and a sheet member that supports the electrodes, the wiring member, the sensor module, and the connector; and an adhesive sheet that can be attached to a living body while covering the electrode sheet.

## Description

### [Technical Field]

The present invention relates to an electrode device for use with a living body.

### [Background Art]

Recently, medical treatment has developed to a high degree, and analyses of body fluids of a patient, such as blood, saliva, and urine, can ascertain the state of health of the patient. Research being conducted includes, for example, determining the presence or absence of dental decay by measuring the pH of saliva, and diagnosing diabetes by measuring the glucose level of tears. These examinations are conducted, for example, by a medical institution measuring and analyzing a body fluid collected by a patient himself/herself.

On the other hand, devices are also being developed that allow a patient to measure and analyze their body fluids by themselves without visiting a medical institution. This device not only enables acceleration of examination and analysis but also is usable as a means of reducing medical costs in an aging society as described below.

Generally, a person has a checkup with a medical institution after they recognize their poor physical condition. However, the person is sometimes terminally ill at that stage. In this case, advanced medical treatment and administration of expensive drugs are conducted, consequently increasing the burden of medical expenses.

If a person can detect their poor physical condition or the like earlier, the person can be cured by milder treatment, such as reviewing their lifestyle, which does not require drug administration or the like. Therefore, there are an increasing number of institutions that conduct routine health checkups as a preventive therapy organized by a health insurance association.

However, a routine health checkup is generally conducted about once or twice a year, and therefore, there is a gap between checkups, and disease that has developed during this period cannot be identified. Thus, the existing preventive therapy has limitations.

If a person uses a device that enables them to measure and analyze their body fluid without visiting a medical institution, examination can be conducted frequently. Therefore, a person can detect a change of their physical condition before otherwise becoming aware of the change. Accordingly, such a device decreases opportunities that require advanced medical treatment and administration of expensive drugs, enabling reduction of medical costs.

As a method for obtaining information on a body fluid, there is a method for attaching a biological sensor to the skin or mucosa and acquiring information of a body's internal environment in-vivo (within the living body). This method enables acquisition of biological information without a time lag, but due to the sensor being brought into direct contact with a living body, the living body is more strongly affected and the sensor is required to have high reliability as a device.

There are various biological sensing methods, and an electrochemical method is widely used, for example, in the measurement of the glucose level, for the reason that a trace component can sensitively be detected. Since the electrochemical method can detect biological information, which is chemical characteristics, as an electrical signal, the electrochemical method has an advantage of facilitating a process and an analysis of a signal obtained using a semiconductor device or the like. Therefore, active development is globally underway for a new electrochemical sensing device and a sensing method using the device.

On the other hand, administration of a medicine to a living body, or, for example, suction or extraction of blood from a living body is also conducted by: attaching, to the epidermis of the living body, a needle-shaped member including, on a substrate thereof, one or a plurality of fine needle-shaped projections (for example, microneedles) that can puncture the skin; and thus puncturing the stratum corneum which is the outermost layer of the skin. Needle-shaped members which have various sizes and shapes have been proposed, and are expected as a non-invasive administration method or examination method.

By puncturing, with the needle-shaped member, the epidermis which is a layer under the stratum corneum, and further the dermis which is a layer under the epidermis, and thereby making the tip portion of the needle-shaped member reach those layers, it can act on those layers. In addition to the administration of a medicine, or suction or extraction of blood from a living body, there are proposals for: obtaining information of a skin lower layer by applying an electrical signal and receiving a response with use of the needle-shaped member (or a probe) as an electrode; and applying a voltage between electrodes for medication.

For example, following Patent Literatures 1 and 2 describe a technique relating to a biological sensing device including a needle-shaped member.

Patent Literature 1 discloses a medical device for diagnosing the disease state of skin of a subject. This medical device includes a conductive probe including a plurality of electrodes, and each of the electrodes includes a plurality of microneedles. In this configuration, each of the electrodes includes a base substrate formed of a silicon substrate. The microneedles are formed integrally with this substrate and disposed horizontally with gaps, and have a length long enough to penetrate the stratum corneum. The microneedles are each configured to have an at least partially slanted shape. Further, the invention described in Patent Literature 1 relates to an electrode for this device, an alignment of the microneedles, and a method for diagnosing a biological state, using impedance measurement. This diagnosis method particularly relates to cancer, particularly skin cancer such as basal cell cancer, a malignant melanoma, and squamous cell cancer, or precursors of such lesions.

Patent Literature 2 discloses a biological monitoring device. Patent Literature 2 describes using microneedles and a flexible interface, and discloses a technique relating to electrocardiogram, sleep assessment, bruxism assessment, sleep apnea, and traumatic brain injury.

A current direction of biological monitoring faces a problem that without measurement pretreatment such as removing the stratum corneum of a subject, the contact impedance between the skin and an electrode cannot be reduced and a highly accurate bioelectrical signal cannot be acquired in brain-wave measurement. This problem causes psychological and physical stress to a subject who wants to detect early signs of disease such as dementia and epilepsy, but no effective countermeasures have been implemented to date. If psychological and physical stress can be reduced to the minimum, there is a merit that simple, accurate, and stress-free brain-wave monitoring can be conducted. Further, if an electrode can more simply be worn directly on the stratum corneum or subcutaneous muscle layer, it will be possible to perform biometric monitoring of things like brain waves while also conducting electrical treatments and other treatments in real time by passing an electric current through the electrodes and checking the effects.

### [Citation List]

### [Patent Literatures]

[PTL 1] JP 5001950 B
[PTL 2] JP 6606067 B

### [Summary of the Invention]

### [Technical Problems]

When a bioelectrical signal from the skin is measured, noise is sometimes generated in the measured wave shape. Due to the influence of this noise, it becomes difficult to check the wave shape, which is to be properly checked, and the measurement is adversely affected in some cases. Since the cause of noise is considered to be resistance of the stratum corneum of the skin, the electrical resistance attributed to the stratum corneum is required to be lowered. Another problem is how closely the skin, an electrode, and a sensor module are attached to each other, because when a bioelectrical signal obtained from the skin is transferred from an electrode toward a sensor module, noise is generated also due to the moment of force generated by the motion of a person, or poor connection or attachment between the sensor module and the electrode.

Generally considered aspects such as Patent Literature 2 employ rivet-type stud means (button top, snap mutual connection). In the rivet-type stud means, an electrode to be in contact with the skin is attached with a base material including an adhesive layer or adhesive agent which adheres to the skin, a conductive gel, or the like, and a rivet-type stud is provided so as to penetrate the base material in the thickness thereof for electrical continuity to a sensor module disposed on the side other than the skin side of the base material attached, that is, on the other surface of the base material on which no adhesive layer, adhesive agent, conductive gel or the like is provided. This configuration facilitates replacement of a disposable attachment-type electrode patch attached to the sensor module.

However, easy detachment of the electrode patch from the sensor module means unstable fixation between the electrode patch and the sensor module, and instability or the like of the sensor module increases the risk of noise generation. Not only the rivet-type stud means, but also an aspect including a position of the electrode in contact with the skin, and a position of the sensor module away from the surface of the skin by the thickness of the base material easily causes a swing of the sensor module in terms of the moment of force and has a high risk of noise generation.

Further, it is clear that, for example, by employing the rivet-type stud means, the sensor module, the electrode, wiring, and the like are present in a mixed state on the front and rear surfaces of the base material, leading to an increase of costs of rivet-type studs, laminate materials, and production steps, and thus giving a great disadvantage for achievement of the disposable attachment-type.

On the other hand, from the viewpoint of wearability, it is essential to attach the electrode and the module to the skin of a person, but firmer attachment makes the person feel a stronger wearing sensation, that is, greater discomfort. Accordingly, another problem to be addressed is reducing this discomfort.

That is, in order to solve these problems, an electrode sheet is desired which not only enables lowering the electrical resistance attributed to the stratum corneum by disposing the above-described needle-shaped electrode or conductive probe including a plurality of electrodes, but also achieves: transferring a bioelectrical signal extracted through the needle-shaped electrode or the conductive probe including a plurality of electrodes to a sensor module while keeping the resistance low; easily conducting multiple-point measurement necessary for brain-wave measurement; and simultaneously solving problems of perspiration that occurs in the interface between the skin of a subject having an electrode attached thereto and the electrode attached, the ability to follow the movement of the skin, and a feeling of unity with the sensor module. Such an electrode sheet is still under development.

Particularly, when it comes to inexpensive electrode sheets, productivity needs to be considered, and the production thereof has no choice but to depend on a printing technique using a conductive paste, such as silver, carbon, and PEDOT, for wiring from the needle-shaped electrode or the conductive probe including a plurality of electrodes to the sensor module.

However, so-called stretchable printed wiring that is low-resistance and stretchable have limitations in the printable height of a single wiring, and the printing line width needs to be expanded to maintain low-resistance wiring. Accordingly, an electrode sheet that only detects a bioelectrical signal with only a few electrodes and transfers the bioelectrical signal to a module has no particular problem, but when it comes to an electrode sheet for performing multiple-point measurement necessary for brain-wave measurement and the like, the electrode sheet has a large area due to the large printing line width thereof and is presumed to cause, for example, situations in which the electrode sheet cannot be attached to the forehead of a subject, and causes, due to the large area thereof, perspiration in the interface between the skin and the electrode sheet, giving discomfort.

There have also been problems of the needle-shaped electrode in which the whole size of the needle-shaped electrode is several millimeters due to processing convenience, and it is difficult to embed the needle-shaped electrode in an electrode sheet and to maintain conductivity on the electrode sheet.

The present invention has been made to solve the problems described above, and an object of the present invention is to provide a structure of an electrode sheet having a low resistance, and to strengthen fixation between the skin, an electrode, and a sensor module and thus reduce noise attributed to the moment of force, and to reduce a wearing sensation felt by a subject having the electrode sheet attached.

### [Solution to Problems]

In order to solve the problems described above, one representative electrode device, according to the present invention, for a living body includes: an electrode sheet including at least a pair of electrodes that receives a bioelectrical signal, a wiring member that transmits the bioelectrical signal received, a sensor module that externally outputs a signal relating to the bioelectrical signal, a connector that connects the bioelectrical signal transmitted to the sensor module, and a sheet member that supports the electrodes, the wiring member, the sensor module, and the connector; and an adhesive sheet that can be attached to a living body while covering the electrode sheet.

### [Advantageous Effects of the Invention]

The present invention provides a structure of an electrode sheet having a low resistance, and can strengthen fixation between the skin, an electrode, and a sensor module and thus reduce noise attributed to the moment of force, and reduce wearing sensation felt by a subject having the electrode sheet attached thereto.

Problems other than described above, configuration, and effects will be clarified by the description in the following Description of the Embodiments.

### [Brief Description of the Drawings]

Fig. 1 shows diagrams each illustrating an electrode sheet according to a first embodiment.
Fig. 2 shows diagrams each illustrating an electrode sheet according to a second embodiment.
Fig. 3 is a diagram illustrating attachment of an electrode sheet to the forehead of a subject.
Fig. 4 shows diagrams each illustrating tight fit.
Fig. 5 shows diagrams each illustrating a method for mounting the first embodiment on a subject.
Fig. 6 shows diagrams each illustrating a method for mounting a third embodiment on a subject.
Fig. 7 shows diagrams each illustrating a conventional wearing method.
Fig. 8 shows diagrams each illustrating a conventional wearing method.
Fig. 9 is a diagram illustrating an electrode sheet according to a fourth embodiment.
Fig. 10 is a diagram illustrating an electrode sheet according to a fifth embodiment.
Fig. 11 is a diagram illustrating an electrode sheet according to a sixth embodiment.
Fig. 12 shows diagrams each illustrating an electrode sheet according to a seventh embodiment.
Fig. 13 is a diagram illustrating a step of removing a mold after printing a wiring member.
Fig. 14 is a diagram illustrating a step of forming a through hole using a punch after printing a wiring member.
Fig. 15 is a diagram illustrating an electrode sheet according to an eighth embodiment.
Fig. 16 is a diagram illustrating a step of producing the electrode sheet according to the eighth embodiment.
Fig. 17 is a diagram illustrating a step of producing the electrode sheet according to the eighth embodiment.
Fig. 18 shows diagrams each illustrating a step of producing the electrode sheet according to the eighth embodiment.
Fig. 19 is a diagram and a photograph illustrating an electrode of the eighth embodiment.
Fig. 20 is a diagram illustrating an electrode according to a ninth embodiment.
Fig. 21 shows diagrams each illustrating the electrode sheet according to the eighth embodiment.
Fig. 22 is a diagram illustrating an electrode sheet according to a tenth embodiment.
Fig. 23 is a diagram illustrating the electrode sheet according to the tenth embodiment.
Fig. 24 is a schematic diagram illustrating an electrode sheet according to an eleventh embodiment.
Fig. 25 is schematic diagrams each illustrating fixation of an electrode in the electrode sheet according to the eleventh embodiment.
Fig. 26 shows schematic diagrams each illustrating fixation of a module or terminal in the electrode sheet according to the eleventh embodiment.

### [Description of the Embodiments]

Hereinafter, embodiments of the present invention will be described with reference to the drawings. However, the present invention is not to be limited by these embodiments. Identical components in the drawings are given identical reference signs.

The present embodiments have a structure for closely attaching, to the skin of a person, a conductive electrode that directly or indirectly extracts an electrical signal generated in the living body, and a sensor module.

In the present embodiments, the electrode and the sensor module are disposed and mounted on the identical surface of an electrode sheet, or on one side of an electrode sheet, and can be fixed with an adhesive sheet while being in contact with the surface of the skin. The mounting on one side can reduce materials and save production steps, leading to reduction of costs.

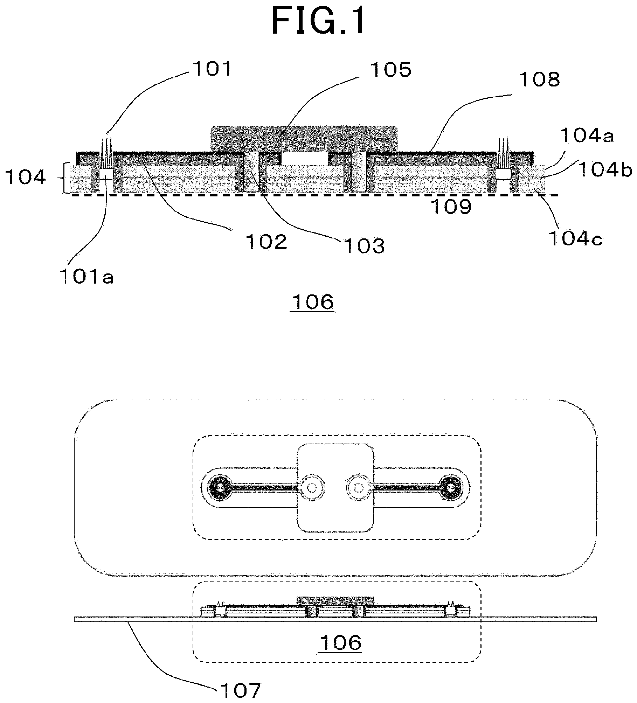

As illustrated in Fig. 1, the present embodiment includes an electrode sheet 106 that includes
a needle-shaped member 101 formed of at least a pair of electrodes that is brought into contact with a living body and receives a bioelectrical signal therefrom,
a wiring member 102 that transmits the voltage of the bioelectrical signal received, and a connector 103 connected to a sensor module 105 that outputs the voltage of the bioelectrical signal to the exterior,
the electrode sheet 106 integrally including the needle-shaped member 101, the wiring member 102, an insulating film 108, the connector 103, a sheet member 104, and a sensor module 105 bonded to the skin and supported by an adhesive sheet 107 as the uppermost layer while the adhesive sheet 107 entirely covering the electrode sheet 106, the needle-shaped member 101 and the sensor module 105 become in contact with the living body, and at least the wiring member, the sheet member, and the adhesive sheet as the uppermost layer have stretchability and elasticity.

Specifically, in the electrode sheet 106 illustrated in Fig. 1, two connectors 103 symmetrically project from the sensor module. To each of these two connectors, the voltage of a bioelectrical signal received from the needle-shaped member 101 is connected via the wiring member 102, and the sensor module receives the voltage obtained. Generation of noise caused by fluctuations of the sensor module due to the motion of exercise or the like of the living body or by instability of the sensor module can further be suppressed along with a feeling of better integration between this reception route from the needle-shaped member 101 to the sensor module and the skin of the living body.

Therefore, the electrode sheet 106 is formed such that the tip of the needle-shaped member 101 and the sensor module 105 of Fig. 1 are positioned close to the surface of the skin to closely attach the sensor module 105 and the needle-shaped member 101 to the skin, and after the electrode sheet 106 is attached to the surface of the skin of the living body, the adhesive sheet is attached to the surface of the electrode sheet 106 opposite to the tip of the needle-shaped member 101 and the sensor module 105 while covering the whole of the electrode sheet 106 and the sensor module 105. The area ratio of the adhesive sheet to the electrode sheet 106 and sensor module 105 should be 5 to 6 or more when the area occupied by the electrode sheet 106 and sensor module 105 is defined as 1, which ensures stable fixation of the electrode sheet 106 and sensor module 105.

### (Needle-shaped member)

The needle-shaped member 101 may be a needle-shaped member originally having conductivity itself, or a needle-shaped member having the entire surface thereof coated with a conductive layer.

In the needle-shaped member 101, a needle-shaped projection (hereinafter, simply called a "needle") is disposed so as to be capable of pressing or puncturing the skin of a patient. This pressing or puncturing reduces the electrical resistance of the skin. In the needle-shaped member 101, a needle having a cross-section in the order of several tens of µm is disposed so as to be capable of puncturing living tissue without causing severe injury.

The needle-shaped member 101 preferably includes a support 101a for disposing the needle thereon. The needle-shaped member 101 is desired to include a plurality of needles projecting upward from the support 101a, and the needles are preferred to have a length in the range of 50 microns to 300 microns, and further, have an outer diameter of the needle outer periphery in the range of 10 microns to 250 microns. The length of the needle and the outer circumference of the needle may be more than 300 microns, e.g., more than 800 microns, because the length of the needle may be insufficient depending on the method of fixing the 101 needle-shaped member, and the associated outer circumference of the needle may be more than 250 microns.

The needles of the needle-shaped member 101 may have various shapes and forms. For example, the distal tip of the needles may be pointed or unpointed, and the needles can have a diagonally cut form, a parabolic form, a tip portion-flattened form, a tip portion-pointed form, a tip portion-unpointed form, a tip portion-rounded form, a tapered form, and/or a tapered circular-cone form. The needle-shaped member may be provided as a multi-dimensional array in contrast with a tool including a single needle or needles forming a single line.

The needle-shaped member 101 can include a form obtained by combining the area, direction, height, or other parameters of the needles. For example, along with an increase of the surface area of the conductive layer, the needle-shaped member 101 increases the effective surface area thereof as an electrode, and the electrical resistance of the skin can be reduced. When the needle-shaped member 101 is made to easily puncture the skin and easily reduce the electrical resistance, the area of the conductive layer can be made small. The conductive layer preferably has a thickness of about 0.05 µm to 5 µm. A conductive layer which is thinner than this range will not maintain conductivity, and a conductive layer which is thicker than this range may possibly impair adhesion to a base material.

The needle-shaped member 101 may be made by known microfabrication processes, by making small mechanical structures out of metals, polymers and other materials.

These microfabrication processes are based on established methods used to make integrated circuits, electronic packages and other microelectronic devices, supplemented with additional methods used in the field of microfabrication.

Examples of microfabrication processes that can be used in the production of the needle-shaped member 101 disclosed in the present description include lithography, etching techniques such as wet etching, dry etching, and photoresist removal, electric plating and non-electrode plating, diffusion processes such as boron diffusion, phosphorus diffusion, arsenic diffusion, and antimony diffusion, ion implantation, film deposition such as vapor deposition (a filament, an electron beam, a flash, and shadowing step coverage), sputtering, chemical vapor deposition (CVD), and epitaxy (gas phase, liquid phase, and molecular beam), electroplating, screen printing, lamination, stereolithography, laser machining, and laser ablation (including projection abrasion).

The needle-shaped member 101 is made from a conductive material belonging to the range described below, and examples of the conductive material include metals, ceramics, semiconductors, organic materials, polymers, and composite materials. However, the conductive material is not limited to these examples. Examples of preferable materials include nickel titanium alloy, medical stainless steel, gold, titanium nickel, iron, gold, platinum, tin, chromium, copper, alloys of these materials or other materials, silicon, silicon dioxide, capacitive carbon, graphite, and polymers. Specific examples of the polymer include highly-conductive PEDOT:PSS (a composite of poly(3,4-ethylenedioxythiophene) (PEDOT) and polystyrene sulfonic acid (PSS)). As an ideal embodiment, materials that are conductive and biocompatible, for example, a nickel titanium alloy, titanium or medical stainless steel, silver/silver chloride, and silver chloride, may be used.

Further, a molded article of the needle-shaped member 101 can be produced by a transfer molding technique, using the needle-shaped member 101 as an original and a duplicate as an intermediate. As one molding method, thermal transfer molding, soft lithography molding, or injection molding may be used. By further coating a transfer molded article with the conductive material described above, a needle-shaped member including the transfer molded article as a base material and having a conductive surface can also be obtained. The molded product of the needle-shaped member obtained by transfer molding may be desirably selected from among polymers that ensure biosafety when puncturing the skin.

For example, polycarbonate, polystyrene, an epoxy resin, polyethylene, polymethyl methacrylate, or polyglycolic acid may be used.

In addition to the needle-shaped member 101 exemplified above, the needle-shaped member 101 may include a shaft having a circular cross-section when viewed perpendicularly, or may be non-circular in cross-section. The cross-section of the needle-shaped member may be, for example, polygonal (for example, a star shape, a square, a triangle, and a circle), oval, or of another symmetric or asymmetric shape, or the needle-shaped member may instead have a structure including a pseudo pyramid (a shape which is similar to a pyramid having a pointed or partially concave tip), a cone, a polyhedron, a pyramid, or a rectangular column (prism).

In most embodiments, the needles are preferred to be directed perpendicular to the support 101a or at another angle. Preferably, the needles are directed to the support 101a perpendicularly so as to increase the density of the needles per unit area of the support 101a. However, the needles are preferred to include combinations of various directions, heights, or other needle parameters. Further, the needles may be identical or non-identical in length.

The needles may have a specific needle density (the number of needles in a specified region). For example, a useful range of separation distance between the needles is 100 to 1400 microns, more preferably 100 to 400 microns. The outer diameter and the length of the needles described above are also important, and it is very important whether a combination thereof with the separation distance enables the needles to actually puncture the stratum corneum of the skin. In one embodiment, the needles have a needle density of at least about 10 needles/cm², more preferably at least about 200 to 2000 needles/cm². These needle densities contribute to uniform depth at which the needles puncture the skin.

In the present embodiment, the needles may have flexibility so as to be capable of fitting themselves to the outline shape of a biological barrier, for example, the skin, to which the needle-shaped member is to be applied. The puncture of the skin is assumed to be limited in some cases due to variation of the surface to which the electrodes for a living body are attached for example, the surface of the skin of a person not being flat due to wrinkles and body hair. The flexibility may mean causing a distortion of 0.1 mm or more and 5 mm or less when a load of 4 kgf is applied to both ends in a range of 1 cm.

### (Conductive member)

The conductive member used in this embodiment is inexpensive, relative to the needle-shaped member.
While the needle-shaped member acquires a bioelectrical signal by directly puncturing the skin, the conductive member acquires a bioelectrical signal by being loaded on the electrode sheet 106 while replacing part of the needle-shaped member, and by using an electrolyte-containing conductive gel interposed between the skin and the conductive member.

The conductive member is made from a conductive material belonging to the range described below, and examples of the conductive material include a metal, ceramic, a semiconductor, an organic material, a polymer, and a composite material. However, the conductive material is not limited to these examples. Examples of preferable materials include nickel titanium alloy, medical stainless steel, gold, titanium nickel, iron, gold, platinum, tin, chromium, copper, alloys of these materials or other materials, silicon, silicon dioxide, capacitive carbon, graphite, and polymers. Specific examples of the polymer include highly-conductive PEDOT:PSS (a composite of poly(3,4-ethylenedioxythiophene) (PEDOT) and polystyrene sulfonic acid (PSS)). As an ideal embodiment, materials that are conductive and biocompatible, for example, a nickel titanium alloy, titanium or medical stainless steel, silver/silver chloride, and silver chloride, may be used.

### (Wiring member)

A structure of the wiring member of the electrodes, according to the present embodiment, for a living body will be described in detail. The wiring member of the present embodiment is formed using a screen printing method. The present screen printing method is described in detail in the description and the drawings of Japanese Patent Application No. 2021-105169 which is a prior application of the present applicant. However, the present embodiment may be implemented by a printing method other than screen printing methods such as a rotary screen printing method, or a screen printing method other than the printing method described in the description of Japanese Patent Application No. 2021-105169.

The present screen printing method for forming the wiring member of the electrodes, according to the present embodiment, for a living body has been realized this time by using a technique which excels at high aspect-ratio (thickness of printing is large) printing, and which includes a feature described in the description of the prior application Japanese Patent Application No. 2021-105169, i.e., simultaneously filling a via and performing high aspect-ratio wiring printing.

The wiring member that can be used in the present embodiment is a wiring member printed using, as a printing ink, a coating agent containing a mixture (base agent) of a filler with a binder, and an additive immiscible in a main solvent which is a solvent of the binder.

As the filler that can be used for the wiring member, an inorganic filler and an organic filler may be used in the present embodiment. The inorganic filler is classified into a metal and a non-metal. The organic filler refers mainly to a polymer composition.

Examples of the metal include noble metals and base metals. Examples of noble metals include gold, silver, platinum, and palladium. Examples of base metals include iron, copper, nickel, aluminum, lead, zinc, tin, tungsten, molybdenum, tantalum, magnesium, cobalt, bismuth, cadmium, titanium, zirconium, antimony, manganese, beryllium, chromium, germanium, vanadium, gallium, hafnium, indium, niobium, rhenium, and thallium. Gold, silver and copper are particularly useful in the present embodiment. A non-metal refers to elements other than the metals described above. Examples of non-metals include in terms of classification by reactivity: hydrogen, carbon, nitrogen, oxygen, fluorine, phosphorus, sulfur, chlorine, bromine, selenium, iodine, and astatine as reactive non-metals; helium, neon, argon, krypton, xenon, and radon as noble gases; and boron, silicon, germanium, arsenic, antimony, and tellurium as semimetals having non-metal-like chemical characteristics.

Examples of the inorganic filler also include a substance obtained through chemical bonding of a plurality of elements, for example, calcium carbonate, silica, carbon black, graphite, a carbon nanotube, alumina, aluminum nitride, boron nitride, beryllia, barium titanate, lead zirconate titanate, ferrite, CMC (carboxymethyl cellulose), titanium oxide, glass beads, magnesium oxide, hydrotalcite, barium sulfate, titanium oxide, zinc oxide, iron oxide, calcium oxide, magnesium oxide, zeolite, calcium oxide, and magnesium oxide.

Examples of the polymer compound as the organic filler include linear polymer compounds formed in a thread or chain shape as a result of chemically bonding the inorganic filler in the manner of a one-dimensional structure through a chemical reaction such as addition polymerization, condensation polymerization, addition condensation, and covalent bonding, and a network polymer compound having a structure in which the inorganic filler is bonded through a covalent bond or the like in the manner of a three-dimensional structure.

The inorganic filler used for the wiring member is an inorganic compound obtained by chemically bonding copper, silver, silicon, or the like with oxygen, hydrogen, carbon, or the like, and refers to one containing an electrically and thermally conductive metal atom as a base, and is preferably one having particularly a siloxane bond or the like.

Examples of organic fillers that can be used include polymers with urethane bonds such as polyurethane, which are usually produced by polyaddition of compounds with isocyanate groups and hydroxyl groups, and have urethane (-NH.CO.O-) bonds; synthetic resins made of polymers of acrylic acid esters or methacrylic acid esters, such as urethane rubber acrylic resins; polymer compounds with amide bonds (similar to proteins); and the highly conductive polymer compound PEDOT:PSS (a composite of poly(3,4-ethylenedioxythiophene) (PEDOT) and polystyrene sulfonate (PSS)).

The shape of the filler is, for example, spherical, flattened, needle-shaped or polygonal, and the particle size of the filler is preferably 0.1 µm to several tens of µm.

Examples of the binder that can be used for the wiring member include a thermosetting resin, a photocurable resin, and a thermoplastic resin.

The thermosetting resin refers to those having not undergone a chemical reaction of the organic filler, such as addition polymerization, condensation polymerization, addition condensation, and covalent bonding, and examples of the thermosetting resin include resins, such as phenolic resins, amino resins, unsaturated polyester resins, epoxy resins, and silicone rubber, which are relatively low-molecular-weight substances and have a three-dimensional crosslinked structure (network structure) of a polymer due to heating, and which do not soften again after being cured, even when heated.

The thermosetting resins have a wide range of reaction temperatures, from room temperature to high temperatures, and react through addition polymerization, condensation polymerization, addition condensation, covalent bonding, or the like. Regarding, for example, silicone rubbers, which are elastomers, contain, as a main raw material, polyorganosiloxanes (silicone polymers) having, as a polymer backbone (main chain), a siloxane bond (Si-O-) in which a silicon atom and an oxygen atom are alternately arranged. The silicone rubbers can be classified into HTV (High Temperature Vulcanizing Rubber, high-temperature curing rubber), LTV (Low Temperature Vulcanizing rubber, low-temperature curing rubber), and RTV (Room Temperature Vulcanizing rubber, room-temperature curing rubber). An HTV has a curing temperature of 140°C or higher, an LTV has a curing temperature of 40 to 140°C, an RTV has a curing temperature of 0 to 40°C. However, due to diversification of rubbers, the boundary between the HTV and the LTV is disappearing, and rubbers are simply classified into a heat curing type and a room-temperature curing type in many cases.

Further, the rubbers can be classified into a solid millable type (HCR; High Consistency Rubber) and a liquid type depending on the properties of the rubbers before curing, and examples of the liquid type include product groups such as LSR (Liquid Silicone Rubber) and RTV. HCR contains a linear gum having a polymerization degree of about 5000 to 10000, whereas liquid silicone rubber (LSR, RTV) contains, as a main component, a linear polymer having a polymerization degree of about 100 to 2000. Further, the rubbers can be classified into peroxide curing, addition-reaction curing, and condensation-reaction curing depending on the crosslinking mechanism of the rubbers.

The photo curable resin refers to a resin that is polymerized and cured by light having a specific wavelength. In short, the binder refers to a thermosetting resin, a photocurable resin, a thermoplastic resin, and the like not having undergone such a chemical reaction as described above, fixation, or adhesion.

As the binder used for the wiring member, resins having rubber elasticity and porosity can be applied, such as a polyurethane-based thermoplastic elastomer which is a block copolymer having a urethane bond, a thermoplastic polyurethane, a thermosetting urethane elastomer, polyester-based acrylic rubber and polypropylene, an acrylic elastomer containing polyester as a main component, and a thermoplastic elastomer containing a block copolymer of methyl methacrylate and butyl acrylate.

Further preferably, a material containing a siloxane is preferably used. Siloxane refers to a polymer formed by alternating bonds between silicon (Si) and oxygen (O), forming a main backbone of a silicone, called a siloxane bond. Particularly, a silicone elastomer containing a siloxane compound as a backbone is a highly effective factor that enhances a flexible or stretchable feature of an electrode.

The wiring pattern printing ink capable of forming the wiring member usable in this embodiment is defined based on a concept completely different from that of printing ink used for general printing purposes.
In general printing ink, a material having a small difference in the solubility parameter (hereinafter, abbreviated as a SP value) from a binder contained in the printing ink is mixed with the binder and used. Two components having a small difference in the SP value can be easily mixed with each other (high solubility), and therefore can secure, for example, ease of handling of the ink during printing, smoothness of printing material, and adhesion of the ink to printed material.

However, in the present embodiment, by using an additive having a great difference in the SP value from the inorganic filler or the organic filler, or/and the binder contained in the printing ink, in contrast to the theory of general printing inks, the additive can, under filling pressure, seep out as a lubricant.

When a silicone elastomer containing a siloxane compound as a backbone is used as the binder of the printing ink as described above, a water-soluble solvent is preferably used as the additive because the silicone elastomer is water-insoluble. Further, the elastomer, which has rubber elasticity and porosity, has pores thereof infiltrated and impregnated with the water-soluble solvent. By using such a printing ink for printing, the additive having been impregnated seeps out under pressure and shear applied to the printing ink, forms a coating at the interface between the plate and the printing ink, and easily exerts lubricity as a lubricant.

Examples of composition of the printing ink used in the present embodiment include a composition obtained by adding an additive to a base agent containing a conductive material, such as silver and carbon, as a filler, a silicone polymer as a binder; and a main solvent (for example, chain siloxanes such as dimethylsiloxane and dodecamethylpentasiloxane, and cyclic siloxanes such as octadecamethylcyclononasiloxane) or a solvent containing, as a main component, aliphatic hydrogen-based solvents or the like, in addition to normal undecane. In this case, since the binder is water-insoluble, the additive should be water-soluble.

The printing ink used in the present embodiment will be described using, as an example, a mixture of a rubber (binder) with an oil (additive). When a rubber (binder) is mixed with an oil (additive), the rubber becomes swollen. This swelling is due to a phenomenon in which the oil enters between molecules of the rubber. When the oil (additive) is miscible with the rubber (binder), the rubber becomes swollen, whereas when the oil is hardly miscible with the rubber, the rubber is hardly swollen, and even if the oil enters between molecules of the rubber (binder), the oil seeps out to the surface of the rubber under, for example, an environment such as compression.

That is, when the binder of the printing ink is water-insoluble, addition of a water-soluble solvent as an additive forms a state in which the additive, which is the water-soluble solvent, migrates to the surface of the printing ink, because substances having different polarities, i.e., substances having a great difference in the SP value therebetween, are hardly mixable with each other.

When the binder of the printing ink is water-soluble, a water-insoluble solvent is used as the additive. Due to the same mechanism described above, the water-insoluble solvent migrates to the surface of the printing ink, and acts as a lubricant.

As the main solvent and the additive used for the printing ink, a water-insoluble solvent or a water-soluble solvent can be used. As a solvent generally contained in a filling agent and a printing ink used in a printing method or the like, a composition containing a compound (excluding monohydroxystearic acid) represented by a structural formula (1) below can be used.

R1-CH₂-R2 (1)

(In the formula, R1 represents a monohydroxy alkyl group, and R2 represents a carboxyl group (C(=O)OH) or an amide group (C(=O)NH₂).)

Specific examples of the solvent include n-heptane (SP value: 7.3), 2-(2-ethoxyethoxy)ethyl acetate (SP value: 9.0), ethylene glycol monoethyl ether acetate (SP value: 8.8), n-propanol (SP value: 11.8), 1,2,5,6-tetrahydrobenzyl alcohol (SP value: 11.3), diethylene glycol ethyl ether (SP value: 10.9), 3-methoxy butanol (SP value: 10.9), triacetin (SP value: 10.2), propylene glycol monomethyl ether (SP value: 10.2), cyclopentanone (SP value 10.0), γ-butyrolactone (SP value: 9.9), cyclohexanone (SP value: 9.9), propylene glycol-n-propyl ether (SP value: 9.8), propylene glycol-n-butyl ether (SP value: 9.7), dipropylene glycol methyl ether (SP value 9.7), 1,4-butanediol diacetate (SP value: 9.6), 3-methoxybutyl acetate (SP value: 8.7), propylene glycol diacetate (SP value: 9.6), ethyl lactate acetate (SP value: 9.6), ε-caprolactone (SP value: 9.6), 1,3-butylene glycol diacetate (SP value: 9.5), dipropylene glycol-n-propyl ether (SP value: 9.5), 1,6-hexanediol diacetate (SP value: 9.5), dipropylene glycol-n-butyl ether (SP value: 9.4), tripropylene glycol methyl ether (SP value: 9.4), tripropylene glycol-n-butyl ether (SP value: 9.3), undecane (SP value: 15.8), decane (SP value: 15.8), dodecane (SP value: 16.0), cyclohexanol acetate (SP value: 9.2), diethylene glycol monoethyl ether acetate (SP value: 9.0), diethylene glycol methyl ether acetate (SP value: 9.0), diethylene glycol monobutyl ether acetate (SP value: 8.9), ethylene glycol monobutyl ether acetate (SP value: 8.9), methyl acetate (SP value: 8.8), ethyl acetate (SP value: 8.7), propylene glycol monomethyl ether acetate (SP value: 8.7), n-propyl acetate (SP value: 8.7), dipropylene glycol methyl ether acetate (SP value: 8.7), 3-methoxybutanol acetate (SP value: 8.7), butyl acetate (SP value: 8.7), isopropyl acetate (SP value: 8.5), tetrahydrofuran (SP value: 8.3), dipropylene glycol methyl-n-butyl ether (SP value: 8.0), dipropylene glycol methyl-n-propyl ether (SP value: 8.0), dipropylene glycol dimethyl ether (SP value: 7.9), propylene glycol methyl-n-butyl ether (SP value: 7.8), propylene glycol methyl-n-propyl ether (SP value: 7.8), and dimethyl siloxane (SP value: 7.5).

In the present embodiment, the SP value is only a reference for selecting a solvent, and what is important is determining whether the main solvent is water-insoluble or water-soluble, and finding a combination of adding a solvent that is the exact opposite to the main solvent.

As the definition of the water insolubility and the water solubility, definition of water-soluble liquids of group IV hazardous substances is referred to. Group IV hazardous substances are flammable liquids. Group IV hazardous substances are further classified into (a) substances soluble in water (water-soluble) and (b) substances insoluble in water (water-insoluble).

These substances are defined by government ordinance as follows. Water-soluble liquids are those which maintain a uniform appearance when formed into a mixed liquid with pure water of the same volume at one atmosphere and 20°C. Water-insoluble liquids are those other than water-soluble liquids, and separate into two layers when mixed with water. When having a lower specific gravity than water, a liquid forms a water-insoluble layer on a water layer, and when a higher specific gravity, under a water layer. When a water-soluble liquid is mixed with water, no separated layers are formed and the mixture is uniform. Those slightly soluble in water, such as diethyl ether, which is a special flammable substance, and ethyl acetate, which is a class 1 petroleum product, are classified as water-insoluble by the definition.

According to the classification based on the definition described above, examples of water-soluble solvents include 2-(2-ethoxyethoxy)ethyl acetate (SP value: 9.0), ethylene glycol monoethyl ether acetate (SP value: 8.8), n-propanol (SP value: 11.8), 1,2,5,6-tetrahydrobenzyl alcohol (SP value: 11.3), diethylene glycol ethyl ether (SP value: 10.9), 3-methoxybutanol (SP value: 10.9), propylene glycol monomethyl ether (SP value: 10.2), γ-butyrolactone (SP value: 9.9), propylene glycol-n-propyl ether (SP value: 9.8), dipropylene glycol methyl ether (SP value: 9.7), ethyl lactate acetate (SP value: 9.6), ε-caprolactone (SP value: 9.6), tripropylene glycol methyl ether (SP value: 9.4), tripropylene glycol-n-butyl ether (SP value: 9.3), diethylene glycol monoethyl ether acetate (SP value: 9.0), ethylene glycol methyl ether acetate (SP value: 9.0), diethyl ether acetate (SP value: 9.0), tetrahydrofuran (SP value 8.3), dipropylene glycol methyl-n-butyl ether (SP value: 8.0), dipropylene glycol methyl-n-propyl ether (SP value: 8.0), dipropylene glycol dimethyl ether (SP value: 7.9), and propylene glycol methyl-n-propyl ether (SP value: 7.8).

Examples of water-insoluble solvents include undecane (SP value: 15.8), decane (SP value: 15.8), dodecane (SP value: 16.0), triacetin (SP value: 10.2), cyclopentanone (SP value: 10.0), cyclohexanone (SP value: 9.9), propylene glycol-n-butyl ether (SP value 9.7), 1,4-butanediol diacetate (SP value: 9.6), 3-methoxybutyl acetate (SP value: 8.7), propylene glycol diacetate (SP value: 9.6), 1,3-butylene glycol diacetate (SP value: 9.5), dipropylene glycol-n-propyl ether (SP value: 9.5), 1,6-hexanediol diacetate (SP value: 9.5), dipropylene glycol-n-butyl ether (SP value: 9.4), cyclohexanol acetate (SP value: 9.2), diethylene glycol monobutyl ether acetate (SP value: 8.9), ethylene glycol monobutyl ether acetate (SP value: 8.9), methyl acetate (SP value: 8.8), ethyl acetate (SP value: 8.7), propylene glycol monomethyl ether acetate (SP value: 8.7), n-propyl acetate (SP value: 8.7), dipropylene glycol methyl ether acetate (SP value: 8.7), 3-methoxybutanol acetate (SP value: 8.7), butyl acetate (SP value: 8.7), isopropyl acetate (SP value: 8.5), propylene glycol methyl-n-butyl ether (SP value: 7.8), and dimethyl siloxane (SP value: 7.5).

When the total weight of the filler and the binder contained in the printing ink used in the present embodiment is defined as 100 parts, the content of the filler may be 50 to 99.9 parts. Specifically, a silver paste, a copper paste, or the like used for, for example, a filling agent or printing uses may be used as a base agent.

When the total weight of the filler and the binder contained in the coating agent is defined as 100 parts, the content of the additive in the printing ink is preferably adjusted in the range of 0.1 parts to 50 parts.

In the printing ink used in the present embodiment, a compatible solvent having good affinity for the binder may be present. The presence of the compatible solvent secures adhesion between the mixture (base agent) of the inorganic filler or the organic filler with the binder, and printed matter, conductivity, continuity, and the like. Lubricity can be obtained while those properties are maintained. When the addition amount is out of the range described above, a chemical bond of an elastomer or the like which is the base agent is damaged and broken, and the elastomer becomes physically brittle, making printing difficult. Even if printing is performed, elastic force may be lost or conductivity or thermal conductivity may be remarkably lost.

The printing ink used in the present embodiment has an action in which a lubricant in the coating agent seeps out under application of pressure to the printing ink in squeegee sweeping. Accordingly, peeling of the coating agent from the mask interface is promoted, and the coating agent is, without causing cohesive failure, transferred to a base material, giving a wiring member having a substantially rectangular cross-sectional shape, with opposite side surfaces thereof substantially vertically upright. However, in the present embodiment, the cross-sectional shape is not necessarily substantially rectangular, and the opposite side surfaces do not have to be substantially vertically upright.

### (Sheet member)

A flexible sheet or stretchable sheet used as the sheet member on which a wiring pattern of the wiring member of the present embodiment is printed will be described.

The flexible sheet or stretchable sheet that can be used in the present embodiment is formed of OPP (oriented polypropylene), CPP (cast polypropylene), HDPE (high-density polyethylene), MDPE (middle-density polyethylene), LDPE (low-density polyethylene), L-LDPE (linear low-density polyethylene), PET (polyethylene terephthalate), PEN (polyethylene naphthalate), O-NY (nylon), PA (polyamide), EVAC (EVA resin), PVC (polyvinyl chloride), SAN (AS resin), ABS (ABS resin), PMMA (methacrylic resin), PVAL (polyvinyl alcohol), PVDC (vinylidene chloride resin), PC (polycarbonate), POM (acetal resin), PBT (polybutylene terephthalate), PTFE (fluororesin), PF (phenolic resin), MF (melamine resin), UF (urea resin), PUR (polyurethane), EP (epoxy resin), UP (unsaturated polyester resin), PS (polystyrene), KOP (polyvinylidene chloride-coated OPP), AL (aluminum foil), AOP (PVA-coated OPP/Tohcello), PT cellophane (plain transparent, normal cellophane), MST cellophane (moisture proof cellophane, PVC-coated), K cellophane (PVDC-coated), VM (aluminum vapor-deposited film, transparent vapor-deposited film), a co-extrusion film, nonwoven fabric, NR (natural rubber), IR (isoprene rubber), BR (butadiene rubber), SBR (styrene-butadiene rubber), IIR (butyl rubber), NBR (nitrile rubber), EPM (ethylene-propylene rubber), EP (epoxy resin), EPDM (ethylene-propylene rubber), CR (chloroprene rubber), ACM (acrylic rubber, copolymer of ethyl acrylate and chloromethyl vinyl ether), ANM (acrylic rubber, copolymer of n-butyl acrylate and acrylonitrile), CSM (chlorosulfonated polyethylene rubber), PUR (polyurethane resin), U (urethane rubber), Si (silicone resin), Q (silicone rubber), VMQ (silicone rubber having a vinyl group and a methyl group attached thereto), SR (silicone rubber), FKM (fluororubber, fluorinated vinylidene-based rubber), FPM (fluororubber), EVA (ethylene-vinyl acetate rubber), CO (epichlorohydrin rubber, homopolymer of epichlorohydrin), ECO (epichlorohydrin rubber), T (polysulfide rubber), a copper foil, or the like. The flexible sheet or stretchable sheet can be used in the form of a single layer or a laminate film including two or more layers.

In the following disclosure, when a flexible sheet is referred to, the flexible sheet can also be read as a stretchable sheet unless a specific reason otherwise is mentioned. Also in the present embodiment, the sheet member 104 can optionally have a form of a single layer or a laminate film including two or more layers.

For example, when pattern printing of the wiring member is performed using a urethane-based printing ink, a urethane-based film can be selected as a print front layer, and, for example, a PET film can be selected as a layer under the urethane-based film to maintain stiffness. Here, a primer or a bonding layer may be sandwiched between the urethane-based film and the PET film to improve adhesion therebetween. Further, another type of sheet, a primer, or a bonding layer may optionally be stacked as a layer under the PET film. When pattern printing of the wiring member is performed using a silicone-based printing ink, a silicone-based film can be selected as a print front layer, and, for example, a PET film can be selected as a layer under the silicone-based film to maintain stiffness. Here, a primer or a bonding layer may be sandwiched between the silicone-based film and the PET film to improve adhesion therebetween. Further, another type of sheet, a primer, or a bonding layer may optionally be stacked as a layer under the PET film.

Here, in consideration of ease of handling of a film to be a base necessary for producing a film of the sheet member, handleability during printing or the like, and stain resistance, a protective film may be provided, and the material for the base film may be a PET film, a polyethylene film, or the like, and is not limited.

In the single-layer or laminated sheet member, through holes such as vias and through holes for inserting and fixing the needle-shaped member 101, conductive member 201, and connector 103 are made at necessary locations with a hole punch in the manner described above. These holes have the wiring member filled therein, for example, by the present screen printing method described later, and are formed into conductive vias or through holes.

The opening diameter of these vias or through holes are to have an interference fit with the diameter of the parts of the needle-shaped member 101, the conductive member 201, and the connector 103 which are inserted into the vias or through holes and fixed there. That is, the fit tolerance with respect to the diameter of the parts of the needle-shaped member 101, the conductive member 201, and the connector 103 which are fixed is set to from zero tolerance corresponding to "transition fit" to a negative tolerance corresponding to "tight fit", and thereby the instability of the needle-shaped member 101, the conductive member 201, and the connector 103 is reduced and strong conductivity can be secured. In addition to this state, reliability can also further be improved by applying a conductive adhesive.

The diameter of the vias or through holes may be a diameter derived by deducting, from the diameter of the parts of the needle-shaped member 101, the conductive member 201, and the connector 103 which are fixed, a proportion of minus 0% to minus 99.9%, preferably a proportion of minus 0% to minus 40%, more preferably a proportion of minus 0% to minus 15% the diameter of the parts. Ultimately, the needle-shaped member 101, the conductive member 201, and the connector 103 may have the tip thereof formed into a needle shape for piercing.

Particularly conventional rivet-type stud means (button top, snap mutual connection) used for connection between a sensor module and an electrode, and electrical connection to a sensor sheet via magnetic connection or the like are simple for general-purpose use, but have been one of factors of increasing the costs of materials for a disposable electrode sheet. However, the aspects of the present invention produce an effect of enabling direct insertion of the connector 103 of the sensor module into a via or through hole having conductivity secured therein, and the costs of materials can be reduced, and therefore, the unit price of a disposable patch can be suppressed.

### (Electrolyte-containing conductive gel)

The electrolyte-containing conductive gel used in the present embodiment is obtained by adding a polymer material exhibiting flexibility, plasticity, and adhesion to an electrolyte solution having at least any one of an amino acid, an organic salt, or an inorganic salt dissolved therein, and solidifying the mixture into a gel. As the polymer material exhibiting flexibility, plasticity, and adhesion, a material having a glass transition point equal to or lower than life temperature, and a melting point equal to or higher than life temperature is used, and examples of this polymer material are considered to include acrylic or polyurethane-based materials. The "living temperature" in the present embodiment means a temperature in the range of 0 degrees to 40 degrees.

### (Insulating film)

The insulating film that can be used in the present embodiment is desired to be made from a material not causing degradation of strength, distortion, melting, alteration, and the like in a desired use time. Examples of the material include a sheet made from a polyester resin such as polyethylene terephthalate, polybutylene terephthalate, polyethylene naphthalate, an ethylene-terephthalate-isophthalate copolymer, and polyacrylate, and an unstretched sheet can preferably be used. Other examples of the material include a resin sheet made from a fluororesin, such as polyvinyl fluoride, polyvinylidene fluoride, polyethylene tetrafluoride, and an ethylene-ethylene tetrafluoride copolymer, a polyimide resin, or the like, and include an RTV silicone rubber. A UV-cured silicone rubber or the like can also be used.

These insulating films can be bonded to a desired range by a method such as thermal fusion, dry lamination, and spray coating. Alternatively, these insulating films can coat a desired range by a printing method such as silk screen, gravure printing, flexographic printing, off-set printing, and roll transfer printing. As a resin binder of an ink to be used, for example, an acrylic resin, a polyester resin, a polyimide resin, a silicone resin, or the like can be used. The insulating coating ordinarily has a thickness of preferably about 20 to 300 µm.

### (Connector)

The connector that can be used in the present embodiment is basically connected to or incorporated in the sensor module.

Regarding the material, the connector is made from a conductive material belonging to the range described below, and examples of the conductive material include a metal, ceramic, a semiconductor, an organic material, a polymer, and a composite material, but the conductive material is not limited to these examples. Examples of preferable materials include nickel titanium alloy, medical stainless steel, gold, titanium nickel, iron, gold, platinum, tin, chromium, copper, alloys of these materials or other materials, silicon, silicon dioxide, capacitive carbon, graphite, and polymers. Specific examples of the polymer include highly-conductive PEDOT:PSS (a composite of poly(3,4-ethylenedioxythiophene) (PEDOT) and polystyrene sulfonic acid (PSS)). As an ideal embodiment, materials that are conductive and biocompatible, for example, a nickel titanium alloy, titanium or medical stainless steel, silver/silver chloride, and silver chloride, may be used.

### (Sensor module)

The sensor module 105 used in the present embodiment may have the connector loaded and fixed thereon. Alternatively, the connector may be fixed on the electrode sheet 106 side and inserted into the sensor module 105 side to achieve connection. The outer shape, the function, and the like of the sensor module 105 are not particularly limited.

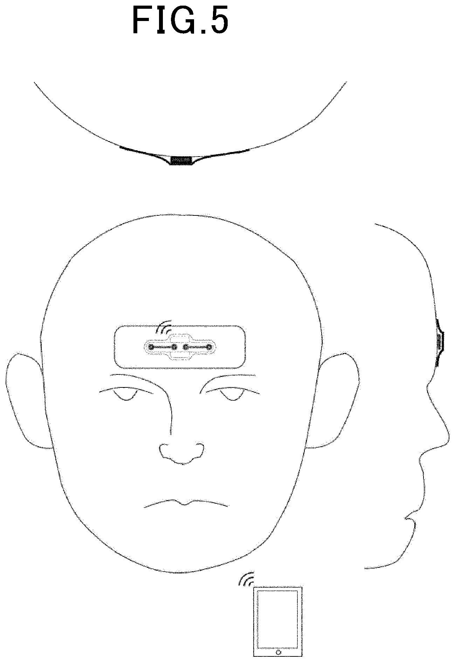

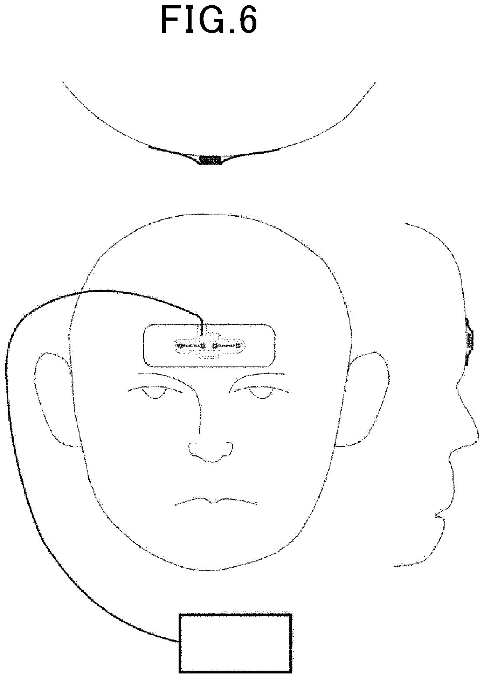

For example, as illustrated in Fig. 5, when the electrode sheet according to the present embodiment is worn on the forehead of a person, the sensor module may have a function of performing wireless transmission and reception between the sensor module end and a mobile phone, a PC, an electric wave transmission and reception base station, or the like. Alternatively, as illustrated in Fig. 6, the sensor module 105 may be connected to a mobile phone, a PC, a measuring device, or the like by wire. In the former case, the sensor module can include a two-dimensional or three-dimensional antenna for radio wave transmission and reception. This antenna may be disposed along or embedded in a wall of a module housing. The antenna may be positioned in the module, or an antenna connected to the sensor module 105 via the connector if necessary and separately printed on the electrode sheet may be used.

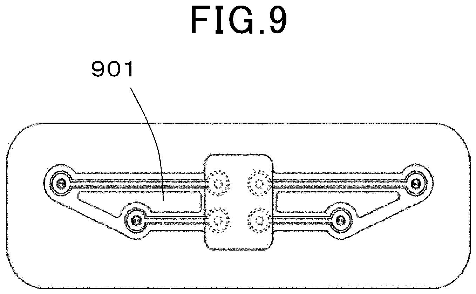

The present embodiment can include connection to a plurality of electrodes via a connector (connection to two or more, for example, four electrodes, connection to an external antenna, or the like) illustrated in Fig. 9. The sensor module may include one signal generator or a plurality of biological amplifiers. This signal generator is configured to provide a signal (for example, a sweep across various frequencies of a plurality of frequencies in the frequency range of, for example, generally between 1 Hz and 10 GHz, between 1 kHz and 10 MHz, between 5 kHz and 1 MHz) between two or more of the electrodes, while the biological amplifiers are configured to capture one or a plurality of signals from two or more of the electrodes. A processor, gate array, digital signal processor, or associated microcircuit is configured to analyze the captured signal to determine the biological impedance of nearby tissue.

The biological amplifiers are configured to capture, from an electrode, a bioelectrical signal (for example, EKG (ElektroKardioGramm), HR (HeartRate), EMG (ElectroMyoGraphy), EOG (ElectroOculoGraphy), EEG (ElectroEncephaloGraphy), and ERG (ElectroRetinoGraphy)). The sensor module can also include a power source (for example, a primary battery, a secondary battery, and an energy harvesting system). Particularly, a power source, such as a flexible thin flexible battery, which is replaceable at opportunities such as replacement of the electrode sheet and reuse of the sensor module, is further preferred. Accordingly, each module may be a self-powered device. The sensor module can include a processor and an internal power source.

The connection may be achieved by wire as illustrated in Fig. 6, enabling reduction of functions of the sensor module 105 and enabling functions such as an antenna and a power source to be externally provided, and therefore, the total weight of the electrode sheet 106 is reduced and discomfort to a subject is reduced.

### (Adhesive sheet member as uppermost layer)

The most distinctive feature of the present embodiment is to dispose an adhesive sheet as the uppermost layer.

Previous bioelectrical signal acquiring electrode sheets have employed a method in which an electrode sheet and a sensor module are separately disposed, and the sensor module is connected to the electrode sheet.

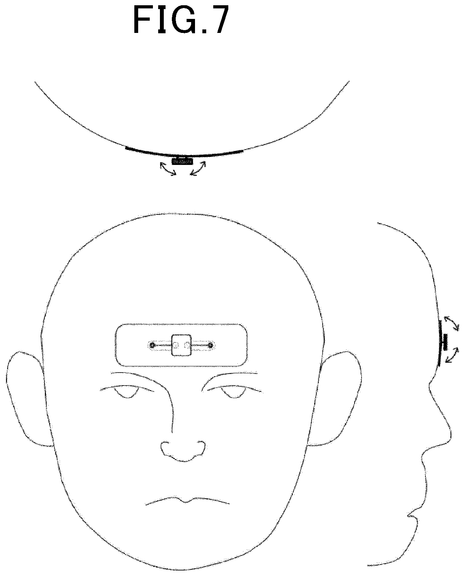

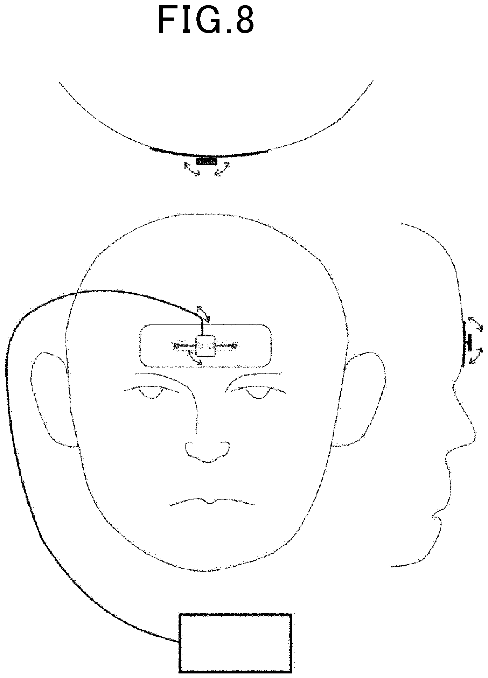

However, a problem is that when a bioelectrical signal obtained from the skin is transferred from an electrode toward a sensor module as illustrated in, for example, the wireless specifications of Fig. 7 and the wired specifications of Fig. 8, noise is easily generated due to an increase in the moment of force generated by the motion of a person, bad connection or bad attachment between the sensor module and the electrode, or a positional relationship therebetween. In addition, in most cases, the electrode sheet and the sensor module are individually independent and worn by overlapping the electrode sheet and the sensor module while a wire is lead out from the electrode sheet, folded back, and connected to the sensor module.

As a result of conducting earnest studies to solve these problems, the present embodiment has employed specifications in which an adhesive sheet is, as the uppermost layer, attached while entirely enclosing an electrode and a sensor module. That is, a feature is the presence of the sensor module on an identical surface with the needle-shaped member 101 and the conductive member 201, which extract a bioelectrical signal, and the wiring member 102 of the electrode sheet, and even when wiring is lead out from the sensor module, noise can be reduced by similarly fixing the wiring with the adhesive sheet. Consequently, the needle-shaped member 101 and the sensor module are in direct contact with the surface of a living body and the moment of force generated by a positional relationship between the electrode and the sensor module is minimized. A basic size of the adhesive sheet member is about 150 mm for width and about 50 mm for length at the largest, and the adhesive sheet can be changed to any shape.

Further, the adhesive sheet can have printing performed thereon with a conductive ink, and pattern printing for the purpose of an electromagnetic shield or decoration, or lamination of a metal foil is also possible.

The adhesive sheet also increases force of pressure attachment of the needle-shaped member or the conductive member to the skin.

This adhesive sheet has a structure of a base material/an adhesive agent, and has the adhesive agent applied to the electrode-and-module-fixation side thereof. The structure of a base material/an adhesive agent is preferred to have a thickness of 10 µm or more and 200 µm or less. The base material contains a urethane resin, may contain at least one selected from the group consisting of an ether-based polyurethane resin, an ester-based polyurethane resin, and a carbonate-based polyurethane resin, and preferably has a thickness of 5 µm or more and 30 µm or less.

The adhesive agent is formed of a synthetic resin, and is preferably formed of a urethane-based adhesive agent. The adhesive layer preferably has a thickness of 5 µm or more and 25 µm or less.

The adhesive sheet formed through lamination has a tensile elongation at break of 130% or more and a tensile stress at 100% elongation of preferably 10 to 100 MPa, more preferably 10 to 30 MPa. The adhesive sheet having a tensile elongation at break and a tensile stress at 100% elongation in this range has an effect of appropriately following, for example, unevenness of the surface of a living body and a stretch and a contraction of the surface of a living body when attached to the living body. The adhesive sheet preferably has a moisture permeability of 2000 g/m²day or more. The adhesive sheet having a moisture permeation in this range has a transpiration effect due to skin respiration, water vapor, or sweating of a living body. The adhesive sheet may be porous. Further, the adhesive sheet may have a slit or a hole made at any position thereof, such as a periphery of the sensor module and a periphery of the electrode sheet, for the purpose of a further improvement of aeration and a ventilation effect on the periphery of the sensor module and the electrode sheet.

A silicone-based material may be used for the adhesive sheet if the same effects can be obtained.

### [Evaluation method]

### [Tensile elongation at break]

The tensile elongation at break was measured by a method in accordance with JIS K 7127:1999 (ISO 527-3:1995) "Plastics-Determination of tensile properties- Part 3: Test conditions for films and sheets" and JIS K 7161-1:2014 (ISO 527-1:2012) "Plastics-Determination of tensile properties- Part 1: General principles". In the measurement of the tensile elongation at break, the adhesive sheet was cut into a dumbbell shape (test piece type 5), and the tensile strength at break was measured using a tensile tester (AGS-X 5kN manufactured by Shimadzu Corporation). The test rate was set to 300 mm/min, the gauge length L₀ was set to 25 mm, and the initial distance L between the fixation tools was set to 80 mm. When the test piece did not have a yield point, a tensile strain at break was calculated as the tensile elongation at break. On the other hand, when the test piece had a yield point, a nominal tensile strain at break was calculated as the tensile elongation at break. In the measurement of the tensile elongation at break, a laminate formed of only a base material and an adhesive agent was used.

### [Tensile stress at 100% elongation]

As in the measurement of the tensile elongation at break, a test piece was prepared in accordance with JIS K 7127:1999 (ISO 527-3:1995) "Plastics-Determination of tensile properties-Part 3: Test methods for films and sheets". Then, the tensile stress at 100% elongation was calculated as a stress when the strain reached 100% in accordance with JIS K 7161-1:2014 (ISO 527-1:2012) "Plastics-Determination of tensile properties- Part 1: General principles".

### (Sheet having photocurable resin applied thereto)

The most distinctive feature of the present embodiment is to dispose an adhesive sheet as the uppermost layer. However, a means of using a sheet having a photocurable resin applied thereto in place of this adhesive sheet as the uppermost layer, and irradiating the sheet having a photocurable resin applied thereto with light while the electrode and the sensor module are closely attached to the skin, to cure the photocurable resin for dry fixation has been found to be also effective to solve the problems of the present invention. Attachment to the skin of a subject is achieved while the whole of the electrode sheet and the sensor module is covered, but a sheet obtained by applying a photocurable resin to a porous film or a PET film is used in place of the adhesive sheet 107.

The photocurable resin refers to a photopolymerizable resin, and may be a bisphenol A-glycidyl methacrylate adduct (Bis-GMA), triethylene glycol dimethacrylate (TEGDMA), urethane dimethacrylate (UDMA), other diacrylates, a simple triacrylate, a mixture of a triacrylate, an inorganic filler such as quartz, silicon nitride, and glass, or a composite material (composite resin) obtained by mixing an organic composite filler or the like containing an organic substance. As a curing light source, a halogen lamp, a xenon lamp, a UV lamp, a visible-light LED, a UV-light LED, or the like may be used. By attaching, in place of the adhesive sheet 107 of the present embodiment, a sheet obtained by applying a photocurable resin to a porous film or a PET film, to the forehead, the head, the top of the head, the body, a hand, or a leg; and irradiating the sheet with a light source for several seconds to several tens of seconds, the photocurable resin was cured and closely attached and held the hair and thereby exerted an anchor effect, and dry attachment to a subject was realized.

After completion of signal measurement, the photocurable resin having been formed into a thin film can be broken with fingers, and the electrode sheet can be removed without great pain during peeling.

### <Electrode sheet>

Hereinafter, the electrode sheet according to the present embodiment example will be described by way of a plurality of embodiments. Needless to say, however, the present invention is not to be limited by these embodiments. In the drawings, components identical with or similar to ones described above are given identical reference signs, and description thereof will be simplified or omitted.

### [First embodiment]

Fig. 1 shows diagrams each illustrating an electrode sheet according to a first embodiment. Hereinafter, the content of the first embodiment will be described according to the production order.

First, as a base material used in the first embodiment, one obtained by laminating together a silicone sheet 104a as an upper layer, an adhesive layer 104b as a middle layer, and a PET film 104c as a lower layer as shown in Fig. 1 was used. In this embodiment, the silicone sheet 104a, the adhesive layer 104b, and the PET film 104c in the laminated state are collectively called a sheet member 104.

Next, a through hole, for inserting a needle-shaped member 101 and a connector 103, having a diameter of 3 mm was made with a punch at four locations. The through holes, made with a punch at the four locations, were disposed at linearly equal intervals, with the center-to-center distance set to 20 mm, 20 mm, and 20 mm. In this embodiment, a PET film was used, but the materials described above, such as polyethylene naphthalate, a urethane sheet, and a polyimide sheet, can also be used to flexibly handle a wiring-forming environment, for example, a heat shrinkage rate.

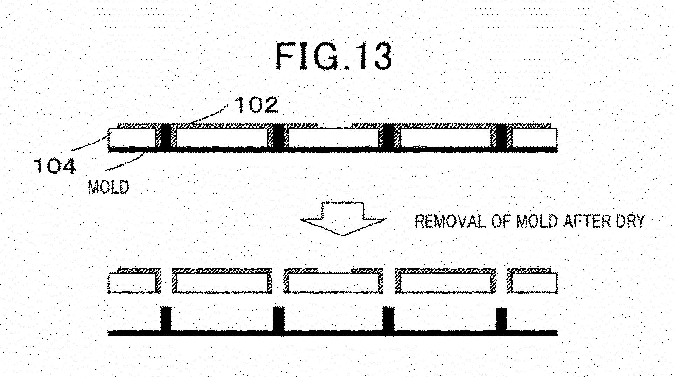

Next, as illustrated in Fig. 13, a metal plate was prepared which had a flat surface thereof joined with the lower bottom surface of a 2.5-mm-diameter metal cylinder disposed at four locations such that the four locations were coincident with the disposition of the four through holes made in the sheet member 104 with a punch at linearly equal intervals of 20 mm, 20 mm, and 20 mm, and the metal plate was superimposed such that the center of the 3-mm-diameter through holes was coincident with the center of the 2.5-mm-diameter cylinders. The 2.5-mm-diameter metal cylinders were inserted into the through holes made in the sheet member 104 with a punch at linearly equal intervals of 20 mm, 20 mm, and 20 mm. The height of the cylinders was coincident with the height of the through holes in the sheet member 104.

This combination of the cylinders with the metal plate is collectively called a mold. In this embodiment, a metal mold is used, but an engineering plastic such as a polypropylene, polyethylene, polyimide, epoxy resin, acrylic resin, silicone, fluororesin, urethane resin, and UV curable resin may also be used.

Next, the wiring members 102 are printed using a technology that simultaneously fills in vias and prints high-aspect-ratio wiring, a feature shown in the specification of Patent Application No. 2021-105169, which is the printing method used in the present application and specialized for high aspect-ratio printing.

The technique described in the description of Japanese Patent Application No. 2021-105169 is a technique of printing a wiring pattern with a conductive coating agent on a sheet which is to be printed and includes a via hole (hole) extending from the front surface side toward the rear surface side, and simultaneously therewith injecting the conductive coating agent into the via hole (hole), and thus connecting, by the conductive coating agent, the front surface side to the rear surface side of the sheet to form a sheet having conductivity on the front surface and the rear surface of the sheet.

By this printing, printing of high aspect-ratio wiring and filling of gaps formed between the 3-mm-diameter through holes made with a punch to insert a needle-shaped member 101 and a connector 103 and the 2.5-mm-diameter cylinders formed in the mold were simultaneously performed with a conductive paste.

Next, the product was dried at 160°C in a thermostatic oven, and had the surface thereof plasma-treated. Thereafter, a PVC silicone rubber was sprayed onto the product as an insulating film and dried.

Next, the mold is removed from the sheet. Fig. 13 is a diagram illustrating a step of removing the mold after printing the wiring member.

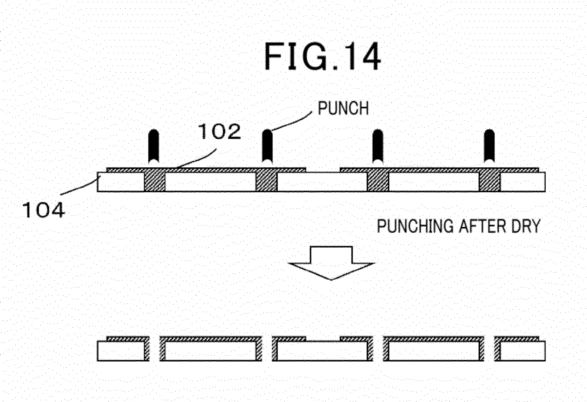

As an alternative method, other than the mold, for forming a through hole, a method using a punch may be used. Fig. 14 is a diagram illustrating a step of forming through holes using a punch after printing the wiring member. This step forms, on the wall surface of through holes, a cylindrical film of the conductive coating agent. Therefore, when a needle-shaped member 101 and a connector 103 are loaded by tight fit in a subsequent step, an effect of facilitating electric connection can be obtained.

Next, while a needle-shaped member 101 was to be loaded, a support 101a needed to achieve tight fit, with a diameter thereof larger than the through holes filled with the conductive coating agent, and therefore, a needle-shaped member 101 with a support having a diameter of 3 mm was selected.

The needle-shaped member 101 was adsorbed with adsorption tweezers in such a manner as not to damage the needle tip, and vertically loaded into the through hole by tight fit, and complete integration of an electrode sheet 106 with the support 101a of the needle-shaped member 101 was confirmed using a continuity check by a tester.

This series of steps complete an electrode sheet excluding a sensor module 105 and a connector 103.

Next, a component which was prepared in advance and in which a cylindrical connector 103 having a diameter of 3 mm and a height of 2 mm was integrated with a sensor module 105 was loaded on the electrode sheet 106.

Two connectors 103 were, by tight fit, effortlessly connected into the two through holes left in the electrode sheet 106.

### (Tight fit)

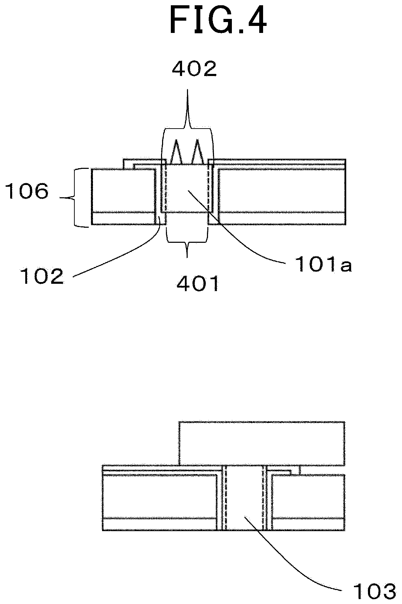

A tight fit refers to a state of insertion in which the relationship between a hole diameter 401 of a through hole and a shaft diameter 402 of the support 101a of the needle-shaped member 101 in the embodiment of Fig. 4 satisfies a relationship of shaft diameter > hole diameter. Due to the electrode sheet 106 according to the present embodiment which is an elastic body, and the elastic force of the wiring member 102 having conductivity, an effect of firmly fixing the needle-shaped member 101 and the connector 103 by tight fit can be obtained. This relation ship is sometimes generally expressed as "interference" or "fit tolerance". The difference in diameter between the shaft and the hole, that is, the fit tolerance, varies depending on, for example, the material of the sheet, and therefore cannot be specified here. However, the hole diameter is to be set to 0 to minus (smaller) with respect to the diameter of the support 101a.

Next, an adhesive sheet 107 including a protective film on the rear surface thereof and having an adhesive layer applied to an attachment surface 109 thereof was prepared, the attachment surface 109 being for attachment to the electrode sheet 106, and the adhesive sheet 107 was attached by disposing the adhesive-layer side of the adhesive sheet on the PET film 104c such that the electrode sheet 106 was positioned in the center of the adhesive sheet 107.

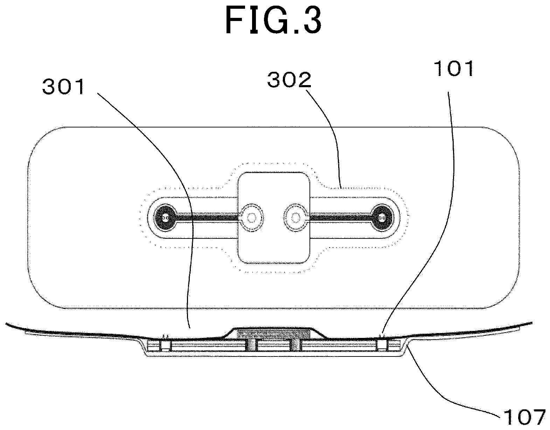

Next, the electrode sheet was uniformly attached to the center of a forehead 301 of a subject as illustrated in Fig. 3, and the protective film on the surface of the adhesive sheet was peeled away. Consequently, a form of the present embodiment was completed in which the adhesive sheet was disposed as the uppermost layer while enclosing the whole of the electrode sheet and the sensor module. A gap 302 at the interface between the forehead 301, the sensor module 105, the electrode sheet 106, and the adhesive sheet 107 may be formed to some degree. A feeling of unity with the forehead 301 of the subject was maintained by extensively covering the electrode sheet 106 and the sensor module 105 with the adhesive sheet. In addition, the maintenance of the feeling of unity enabled stable adhesion of the needles of the needle-shaped member 101 into the stratum corneum of the forehead 301 of the subject.

A specific form enabling stable adhesion of the needles of the needle-shaped member 101 is that the sensor module 105 is disposed in the center of gravity, that is, the center of the adhesive sheet, and in the electrode sheet 106, the wiring member 102 has such a form as to be symmetrically spread in a wing-like manner with respect to the center of gravity (center) of the sensor module, and the needle-shaped member 101 is loaded at a position further by 2 cm or 3 cm rather than 1 cm from the connector 103 of the sensor module 105. This specific form enables influence of the gap 302 of the adhesive sheet on the periphery of the sensor module 105 to be avoided, enabling stable adhesion of the needles. Accordingly, the lower the height of the sensor module 105 from the attachment surface, if set to 2 mm or 1 mm rather than 3 mm, the smaller the gap 302 of the adhesive sheet, enabling stable sticking of the needles of the needle-shaped member 101.

In the first embodiment, the quality of a signal that could be acquired through the connector 103 was evaluated. An object of the evaluation is to find how close the accuracy of the contact impedance transmitted from the needle-shaped member 101 to the connector 103 of the present embodiment approaches the accuracy of a medical needle electrode used in a medical setting.

Two electrodes NE224S manufactured by NIHON KOHDEN CORPORATION and the electrode sheet 106 according to the present embodiment were prepared and mounted on chicken meat (thigh) with skin and subjected to measurement performed by a two-terminal method, using an impedance measuring apparatus (ZM2376 of NF Corporation). Regarding the measured resistance values, the medical needle electrodes had a maximum resistance value of 2.2 KΩ (0.5 Hz), a minimum resistance value of 0.7 KΩ (30 KHz), and a resistance value at around 100 Hz of 1.0 KΩ, whereas the electrode sheet 106 according to the present embodiment had a maximum resistance value of 6.2 KΩ (0.5 Hz), a minimum resistance value of 3.5 KΩ (30 KHz), and a resistance value at 100 Hz of 4.2 KΩ. Two medical gel electrodes (Vitrode F manufactured by NIHON KOHDEN CORPORATION) including an electrolytic solution were measured under the same conditions. The electrodes had, as results, a maximum resistance value of 727 KΩ (0.5 Hz), a lowest resistance value of 2.7 KΩ (30 KHz), and a resistance value at around 100 Hz of 101 KΩ.

Consequently, the measurement of the three types of electrodes by a two-terminal method resulted in the needle electrodes having the smallest resistance values, the electrode sheet according to the present embodiment having values slightly greater than those of the needle electrodes, but much smaller than the values of the gel electrodes. The center frequency in measurement of a bioelectrical signal is about 1 Hz to 200 Hz, and the electrode sheet according to the present embodiment had resistance values much smaller than the values of the gel electrodes up to around 100 Hz.

Accordingly, it has been found that the electrode sheet according to the present embodiment is an effective electrode.

### [Second embodiment]

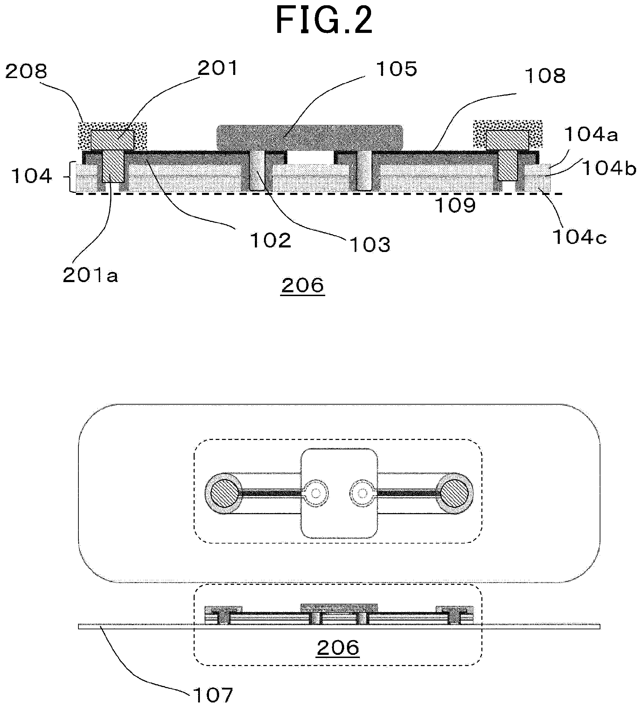

Fig. 2 is diagrams each illustrating an electrode sheet according to a second embodiment. Hereinafter, the content of the second embodiment will be described according to the production order.

First, as a base material used in the second embodiment, one obtained by laminating together a silicone sheet 104a as an upper layer, an adhesive layer 104b as a middle layer, and a PET film 104c as a lower layer in Fig. 2 was used. In this embodiment, the silicone sheet 104a, the adhesive layer 104b, and the PET film 104c in the laminated state are collectively called a sheet member 104.

Next, a through hole, for inserting a conductive member 201 and a connector 103, having a diameter of 3 mm, was made with a punch at four locations.

Next, a metal plate was prepared which had a flat surface thereof joined with the lower bottom surface of 2.5-mm-diameter metal cylinders, and the metal plate was superimposed such that the centers of the 3-mm-diameter through holes were coincident with the centers of the 2.5-mm-diameter cylinders. The height of the cylinders was coincident with the height of the through holes in the sheet member 104.

This combination of the cylinders with the metal plate is collectively called a mold. As the material for the metal plate, stainless steel, nickel, copper, aluminum, iron oxide, graphite, or the like may be used.

Next, the wiring members 102 are printed using a technology that simultaneously fills in vias and prints high-aspect-ratio wiring, a feature shown in the specification of Patent Application No. 2021-105169, which is the printing method used in the present application and specialized for high aspect-ratio printing.

By the printing, printing of high aspect-ratio wiring and filling of gaps formed between the 3-mm-diameter through holes made with a punch to insert a conductive member 201 and a connector 103 and the 2.5-mm-diameter cylinders formed in the mold were simultaneously performed with a conductive paste.

Next, the product was dried at 160°C in a thermostatic oven, and had the surface thereof plasma-treated. Thereafter, a PVC silicone rubber was sprayed onto the product as an insulating film and dried.

Next, the mold is removed from the sheet. Fig. 13 is a diagram illustrating a step of removing the mold after printing the wiring member.

As an alternative method, other than the mold, for forming a through hole, a method using a punch may be used. Fig. 14 is a diagram illustrating a step of forming through holes using a punch after printing the wiring member.

Next, while a conductive member 201 was to be loaded, a support 201a needed to achieve tight fit, with a diameter thereof larger than the through holes filled with the conductive paste, and therefore, a conductive member 201 with a support having a diameter of 3 mm was selected.

The conductive member 201 was adsorbed with adsorption tweezers, and vertically loaded in the through hole by tight fit, and complete integration of an electrode sheet 206 and the support 201a of the conductive member 201 was confirmed.

This series of steps complete an electrode sheet excluding a sensor module 105 and a connector 103.

Next, a component which was prepared in advance and in which a cylindrical connector 103 having a diameter of 3 mm and a height of 2 mm was integrated with a sensor module 105 was loaded on the electrode sheet 106.

Two connectors 103 were, by tight fit, effortlessly connected to the two through holes left in the electrode sheet 206 in the manner of the first embodiment.

Next, a conductive gel 208 was attached while covering the whole of the exposed surface of the conductive member 201.

Next, an adhesive sheet 107 including a protective film on the rear surface thereof and having an adhesive layer applied to an attachment surface 109 thereof was prepared, the attachment surface 109 being to be attached to the electrode sheet 206, and the adhesive sheet 107 was attached in the manner of putting the adhesive-layer side of the adhesive sheet on the PET film 104c such that the electrode sheet 206 was positioned in the center of the adhesive sheet 107.

Next, the electrode sheet was uniformly attached to the center of a forehead 301 of a subject as illustrated in Fig. 3, and the protective film on the surface of the adhesive sheet was peeled away. Consequently, a form of the present embodiment was completed in which the adhesive sheet was disposed as the uppermost layer while enclosing the whole of the electrode sheet and the sensor module.

This embodiment provides a lower-priced version compared to the first embodiment, which is a higher-priced version.

Since a conductive gel is used, the performance is inferior to that of the first embodiment; however, the advantageous feature of this embodiment is that it employs a configuration in which the entire surface is covered with an adhesive sheet, so that it is specialized in providing good attachment of the electrodes and sensor module rather than accuracy of the contact impedance.

The wearability in the first embodiment and the second embodiment was checked by the presence or absence of an unpleasant sensation felt by a subject when the sensor module was placed outside the adhesive sheet and when the sensor module was included in the adhesive sheet, that is, the present embodiment, and checked by the variation of the resistance value measured from a connection part.

Regarding unpleasant sensation experienced by a subject, the feeling of unity of the present embodiment was, needless to say, naturally superior, and the variation of the resistance value was smaller in the present embodiment.

### [Third embodiment]

Following the content of the evaluation performed in the first embodiment, in which the quality of a signal that could be acquired through the connector 103 was evaluated using chicken meat (thigh) with skin, measurement was performed with connection of an impedance measuring apparatus (ZM2376 from NF Corporation) to the sensor module through a conductive wire, while shaking the conductive wire at 1 Hz and an amplitude of 20 cm. The biological electrode device according to the present embodiment had, with substantially no noise, a maximum resistance value of 6.2 KΩ (0.5 Hz), a minimum resistance value of 3.5 KΩ (30 KHz), and a resistance value at around 100 Hz of 4.2 KΩ. In contrast, when the sensor module was subject to external connection and the conductive wire was similarly shaken, the measurement was impossible due to extreme noise.

That is, partially fixing the conductive wire while covering the whole of the electrode sheet and the sensor module with the adhesive sheet at the end was directly linked to the presence or absence of noise, and this result can be said to show the effectiveness of the present embodiment.

From this, it can be said that the same is true for the low-cost specification using the conductive gel of the second embodiment.

Figs. 5 and 6 illustrate a method for mounting the first embodiment and the third embodiment on a subject, and Figs. 7 and 8 each illustrate a conventional wearing method. From the content described above, even when the chicken meat (thigh) is replaced with a human body, the effects of the present embodiment can be said to be more excellent than at least the conventional mounting methods.

### [Fourth embodiment]

Fig. 9 illustrates an embodiment of simultaneously acquiring two or more bioelectrical signals, that is, a plurality of locations, of the electrode sheet according to the first embodiment. Alternatively, Fig. 9 illustrates an embodiment of also including an electrode for electrical therapy which passes an electric current simultaneously with acquiring a bioelectrical signal. The basic production method is not different from the production method of the first embodiment. In the first embodiment, by making a through hole at four locations, two pairs of the needle-shaped member 101 and the connector 103 were formed, whereas, in the present embodiment, the number of through holes made was 8 and four pairs of the needle-shaped member 101 and the connector 103 were formed. Naturally, more through holes can be produced, and, for example, brain-wave measurement may thereby be performed by multiple-point measurement. However, since the area occupied by wiring is increased in the electrode sheet along with an increase in the number of points, a through hole 901 which is not for use of measurement may be provided between wiring lines at various locations in the electrode sheet. This through hole 901 can reduce discomfort caused by perspiration in the interface between the skin of a subject and the electrode sheet.

This embodiment can also be applied to the second embodiment and the third embodiment. For example, an electric current can be passed through the needle-shaped member on the basis of the bioelectrical signal obtained through the conductive gel and the conductive member of the second embodiment. In that case, the wired method of the third embodiment can further increase these effects.

### [Fifth embodiment]

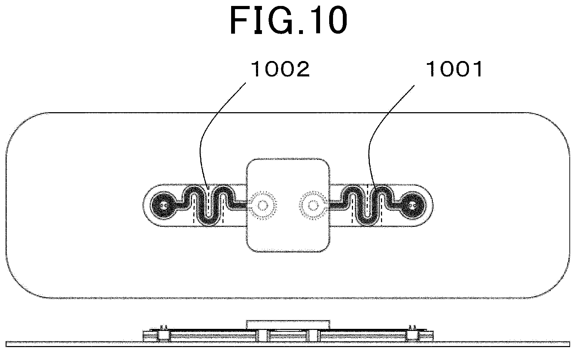

In the first to fourth embodiments, the pattern of the wiring member 102 may be meandering as illustrated in Fig. 10. For example, the wiring member 102 may be continuous wiring having a regularly meandering horseshoe shape. When the wiring member 102 is formed on a flexible and stretchable base material to form an electrode sheet and there is a possibility of noise occurring due to the motion of a subject, undulation of the attachment surface, stretching, shaking, or the like, a meandering pattern 1001 of Fig. 10 may be formed. For more flexible following performance, the electrode sheet may have a slit 1002 formed thereon corresponding to any place of the meandering pattern.

### [Sixth embodiment]

In the first to fifth embodiments, the sheet member 104 may be a single layer or a laminate sheet including two or more layers. The first to fifth embodiments mainly illustrate a sheet member 104 having a three-layer structure and including a PET base material. This PET base material has higher stiffness than the an elastomer material such as a silicone sheet having, on the basis of ASTM standard D638, a tensile strength of 48 MPa to 73 MPa, an elongation at break of 30 to 300%, and a tensile modulus of elasticity of 2800 MPa to 4200 MPa as general performance. Accordingly, by using such a material as a part of the sheet member 104, an advantage of enabling acquisition of a delicate bioelectrical signal, such as brain waves, without greatly distorting the bioelectrical signal can be obtained. The first to fifth embodiments have a configuration assumed to obtain such an effect.

However, for example, even when great distortion is caused by an elastomer such as a silicone sheet and distortion of the bioelectrical signal obtained is large, sufficient measurement is possible for a sheet member 104 configured to include a layer only formed of an elastomer, by establishing, using software, for example, a functional expression of variation in the contact resistance value of the bioelectrical signal with respect to the stretch ratio or the distortion rate of the elastomer used, due to current development of measurement and analysis software.

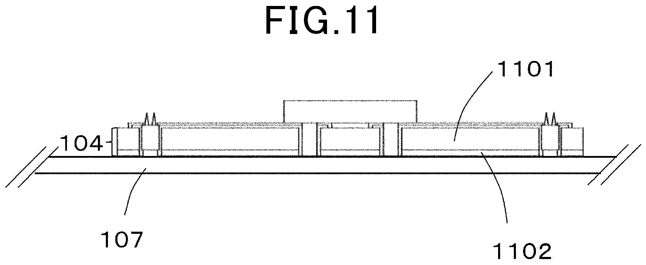

As illustrated in, for example, Fig. 11, the sheet member 104 can also be formed by laminating together the adhesive sheet 107 and a film (elastomer material 1101) such as a silicone sheet, or by directly applying an elastomer to the adhesive sheet 107 by screen printing or the like. The lamination of a film tends to weaken the adhesion between the film and the adhesive sheet 107 because of surface energy, and therefore, a configuration may be employed in which a primer or an adhesive agent 1102 for enhancing the adhesion to the adhesive sheet 107 is applied to a lower layer of the film. In the cases of directly applying an elastomer by printing or the like, a primer may or may not be applied to the adhesive sheet 107 in advance.

### [Seventh embodiment]

In the first to sixth embodiments, the adhesive sheet 107 has, as described in the embodiments above, a form of being attached to the skin of a subject while covering the whole of the electrode sheet and the sensor module. While all of or a part of the data of bioelectrical signals obtained in the sensor module is transmitted as radio waves, the radio waves being emitted from the sensor module, radio waves incident from outside, and other electromagnetic waves, are assumed to affect the electrode sheet 106, 206.

To address this, a solid or mesh pattern printing can be performed on the adhesive sheet 107, by ink-jet printing, using a conductive ink, without covering a part of the sensor module, but covering at least the sheet member 104. The term "solid" is a printing term and refers to a state in which application has been performed on 100% of the entire surface. A mesh pattern is employed when permeability, moisture permeability, designability, and the like are considered. Regarding solid printing, a design such as a pattern may be printed or skin-tone printing that appears similar to the skin may be performed on the adhesive sheet 107 using a conductive ink. This printing is not limited to ink-jet printing, but off-set printing, screen printing, gravure printing, flexographic printing, a dispenser, or a 3D printer may be used. Alternatively, a metal foil of copper, aluminum, or the like having the same effect may be attached. This is a so-called electromagnetic shield.

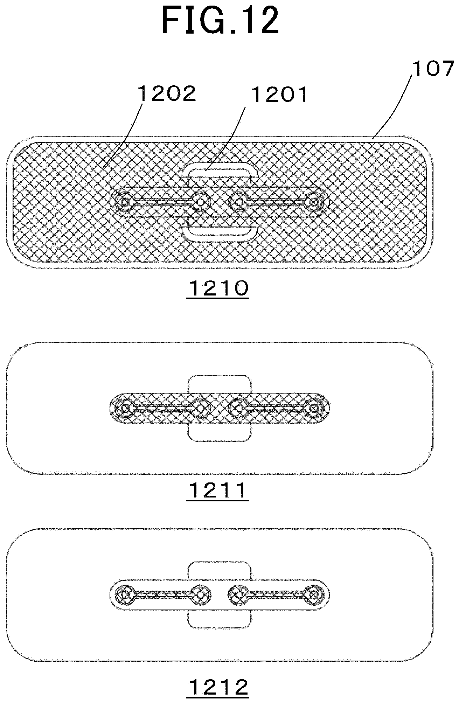

Using this electromagnetic shield, a phenomenon can be avoided in which an essentially unintended electric current generated in the sensor module and the electrode sheet by transmission and reception between the sensor module and the exterior and by an attack of electromagnetic waves to the electrode sheet is induced. Fig. 12 illustrates a mesh pattern applied to the adhesive sheet. A mesh pattern 1202 may be printed on an entire surface 1210 of the adhesive sheet while the pattern is not printed so as not to cover a part 1201 of the sensor module, or the mesh pattern may be printed in a shape 1211 covering only the part of the electrode sheet or in a shape 1212 covering only the part of the wiring member.

The mesh pattern tends to be preferred when it is as unnoticeable as possible, and the mesh pattern is configured to have a reticular shape formed by thin lines having a line width of 30 µm or less, more preferably 15 to 20 µm or less, and a line thickness of 3 µm or less at a pitch of 300 µm to 500 µm. The surface resistivity obtained is 40 Ω/□ or less, more preferably 1.2 Ω/□ or less.

### [Eighth embodiment]

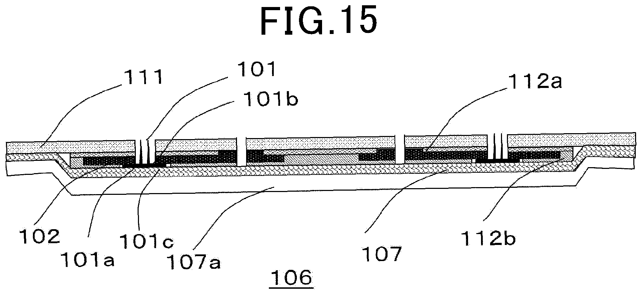

The first to seventh embodiments are achieved by a method for securing conductivity by completely integrating the electrode sheet 106 with the support 101a of the needle-shaped member 101 or the support 201a of the conductive member 201, and the connector 103 through loading thereof by tight fit in vertical through holes in the electrode sheet 106, 206, whereas another method for securing conductivity includes achieving the integration with the electrode sheet 106 and securing conductivity by sandwiching the parts of an upper flat surface 101b and a lower flat surface 101c of the support 101a of the needle-shaped member 101 illustrated in Fig. 15 between layers of the wiring member 102 and the adhesive sheet 107. As the wiring member referred to herein, an ink containing a silver, copper, or carbon filler, or a conductive polymer such as PEDOT:PSS may be used.

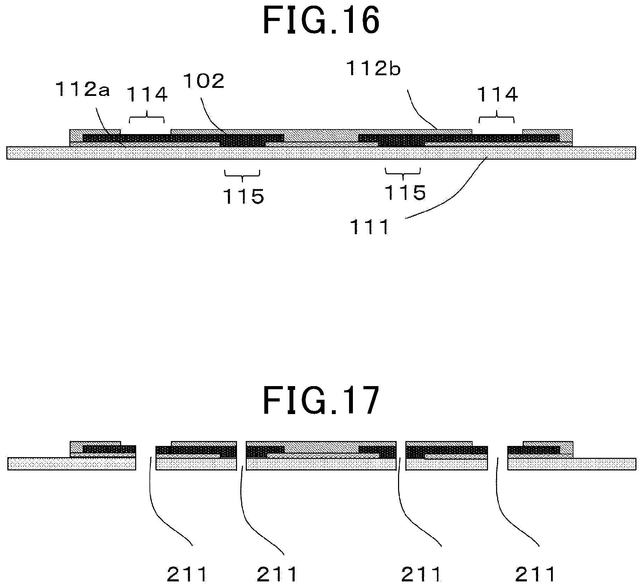

For the base material used in the embodiments, an insulating film 112a is, in Fig. 16, first printed on a release film 111 except in a region 115 into which the connector 103 is inserted in a posterior step. The wiring member 102 is printed thereon. Next, an insulating film 112b is printed except in a region 114 into which the needle-shaped member 101 is inserted later. Drying and curing are performed each time after the printing in the steps described above.

Next, as illustrated in Fig. 17, holes 211 for inserting the needle-shaped member 101 and the connector 103 are made with a punch through the release film, insulating film(s), and the wiring member.

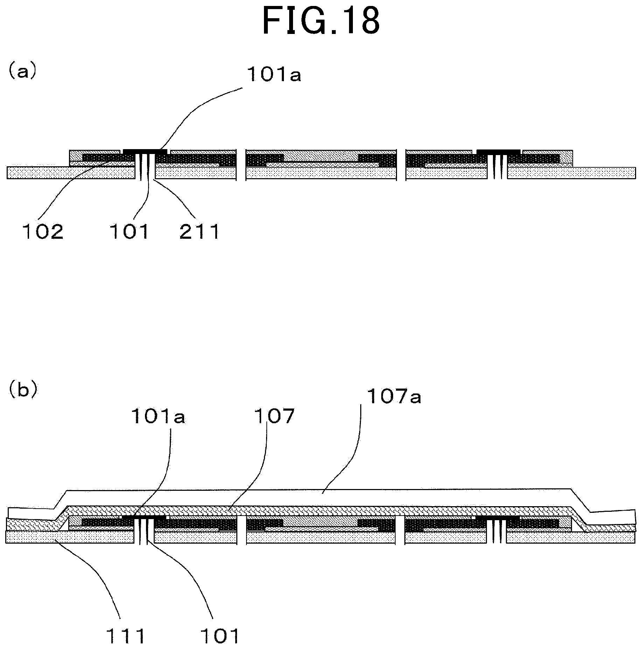

Next, as illustrated in Fig.18(a), the needle-shaped member 101 is inserted into the hole 211 while the support 101a of the needle-shaped member 101 is in contact with the wiring member 102. While this insertion of the needle-shaped member 101 enables continuity between the wiring member 102 and the needle-shaped member 101, a conductive adhesive may be applied to a contact part and the periphery thereof as necessary.

Next, as illustrated in Fig. 18(b), the adhesive sheet 107 is bonded to the entire product including the release film 111. This adhesive sheet 107 has a separation film 107a bonded thereto.

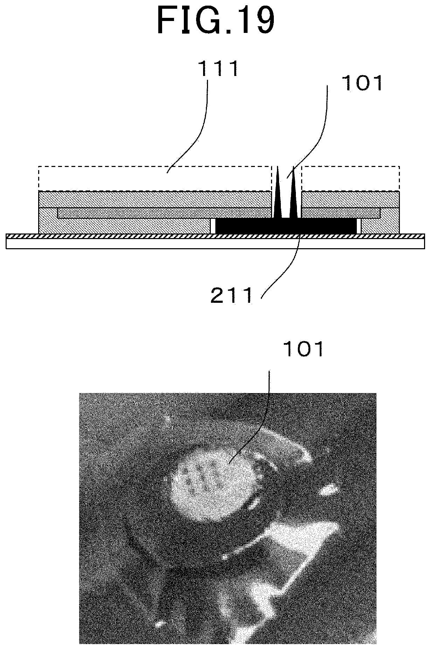

Fig. 19 is a diagram and a picture illustrating exposure of the needle-shaped member 101 of this embodiment from the hole 211 when the release film 111 is peeled, and firm close attachment and fixation of the needle-shaped member 101 to the electrode sheet 106 separated from the release film 111, and illustrates a feature of the present embodiment which realizes a feeling of unity by firmly sandwiching the needle-shaped member 101 between the wiring member 102 formed by printing and the adhesive sheet 107, and allowing only part of the needles of the needle-shaped member 101 to protrude from the hole 211. This feature enhances continuity.

This step forms the electrode sheet 106 in which the release film 111, the insulating film 112a, the wiring member 102, the insulating film 112b, the needle-shaped member 101, the adhesive sheet 107, and the separation film 107a are integrated with each other. Depending on the convenience of handleability or packaging capacity, a sheet having a large area is not used as it is, but may be divided and formed into any shape and size.

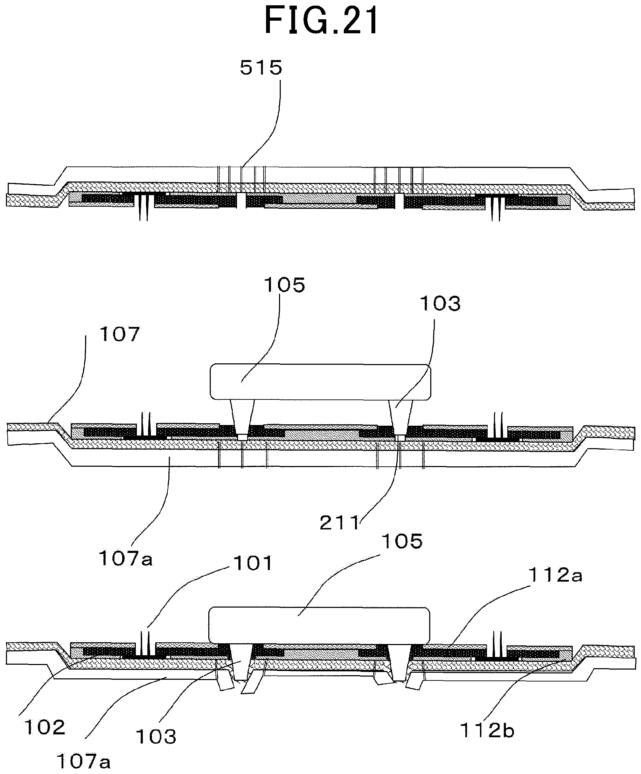

As the adhesive sheet 107 and the separation film 107a used herein, an adhesive sheet and a separation film can also be used a part or the entire surface of which has a plurality of needle holes 515 made therethrough in advance at any intervals as illustrated in Fig. 21.

It is sometimes difficult for a subject to insert the connector 103 of the sensor module 105 into the hole 211 of the electrode sheet 106 due to the pressure of the separation film 107a against the connector 103. Due to the presence of the needle holes 515, the tip of the connector 103 can crush a part of the separation film 107a with the needle holes 515 as a start, enabling closer attachment of the connector 103 to the hole 211. This configuration further secures continuity from the connector 103 to the needle-shaped member 101. An advantage of the needle holes 515 is that the needle holes 515 can be imparted simply by passing, in a production step of the adhesive sheet 107, the adhesive sheet 107 and the separation film 107a through a device including a stretch roll having embossing performed thereon with many pointed needles, on a continuous line such as roll-to-roll processing.

The size and the number of these needle holes 515 can changed arbitrarily, or alternatively, a circular or polygonal aperture can be substituted for the needle holes.

### [Ninth embodiment]

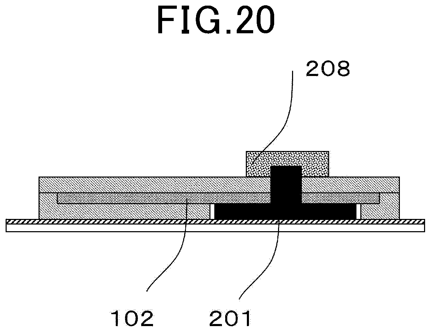

Fig. 20 illustrates use of the conductive member 201 in place of the needle-shaped member 101 illustrated in the eighth embodiment and Fig. 19, and a structure enabling acquisition of a biological signal via not the needle-shaped member 101 but the electrolyte-containing conductive gel 208.

Similarly to the procedure described in the eighth embodiment, a production process includes printing the insulating film 112a on the release film 111 except a region 115 into which the connector 103 is inserted in a subsequent step. The wiring member 102 is printed thereon. Next, the insulating film 112b is printed except for a region 114 into which the conductive member 201 is inserted later in place of the needle-shaped member 101. Drying and curing are performed each time after the printing in the steps described above. Next, as illustrated in Fig. 17, holes 211 for inserting the conductive member 201 in place of the needle-shaped member 101, and the connector 103, are made with a punch through the release film, insulating film(s), and the wiring member. Next, as illustrated in Fig. 20, a part of the conductive member 201 is inserted into the hole 211 while the conductive member 201 is in contact with the wiring member 102. While this insertion of the conductive member 201 enables continuity between the wiring member 102 and the conductive member 201, a conductive adhesive may be applied to a contact part and the periphery thereof as necessary.

Subsequent steps are the same as in the eighth embodiment, but in the ninth embodiment, before supply to the subject side, a step of once releasing the release film 111 and attaching the electrolyte-containing conductive gel 208, and re-attaching a release film is added.

### [Tenth embodiment]

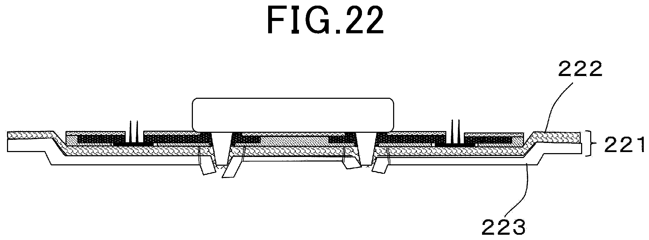

In the first to ninth embodiments, the adhesive sheet 107 has, as described in the embodiments above, a form of being attached to the skin of a subject while covering the whole of the electrode sheet 106 and the sensor module 105. However, as illustrated in Fig. 22, a sheet 221 obtained by applying, to a porous film or PET film 223, a photocurable resin 222 may be used in place of the adhesive sheet 107.

The photocurable resin 222 refers to a photopolymerizable resin, and may be a bisphenol A-glycidyl methacrylate adduct (Bis-GMA), triethylene glycol dimethacrylate (TEGDMA), urethane dimethacrylate (UDMA), other diacrylates, a simple triacrylate, a mixture of a triacrylate, an inorganic filler such as quartz, silicon nitride, and glass, or a composite material (composite resin) obtained by mixing an organic composite filler or the like containing an organic substance. As a light source for curing, a halogen lamp, a xenon lamp, a UV lamp, a visible light LED, a UV-light LED, or the like may be used.

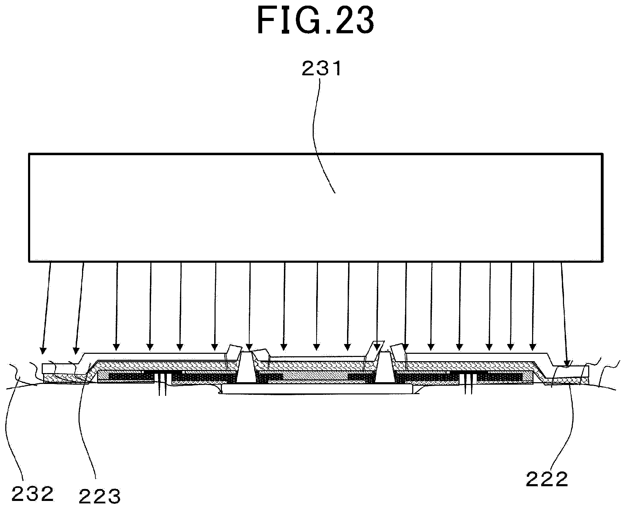

As illustrated in Fig. 23, by attaching, in place of the adhesive sheet 107 of the present embodiment, the sheet 221 obtained by applying the photocurable resin 222 to the porous film or PET film 223, to the forehead, the head, the top of the head, the body, a hand, or a leg; and irradiating the sheet 221 with a light source 231 for several seconds to several tens of seconds, the photocurable resin was cured and closely attached and held the hair 232 and thereby exerted an anchor effect, and dry attachment was realized. The light source 231 may be applied to the sheet 221 while pressed against the sheet 221. The photocurable resin 222 is thereby preferably formed into a film having a small film thickness. Basically, UV-A (wavelength of 315 to 400 nm) is used. While the required cumulative light intensity is preferably in the range of 1 mJ/cm² to 4000 mJ/cm², more preferably in the range of 1 mJ/cm² to 2000 mJ/cm², appropriate light intensity is selected eventually in consideration of influence on the human body.

After completion of signal measurement, the photocurable resin having been formed into a thin film can be broken with fingers, and the electrode sheet can be removed without great pain during peeling.

The tenth embodiment can be applied for fixation of a biological electrode device. Particularly, the tenth embodiment is effective when the needle-shaped member is used as an electrode.

### [Eleventh embodiment]

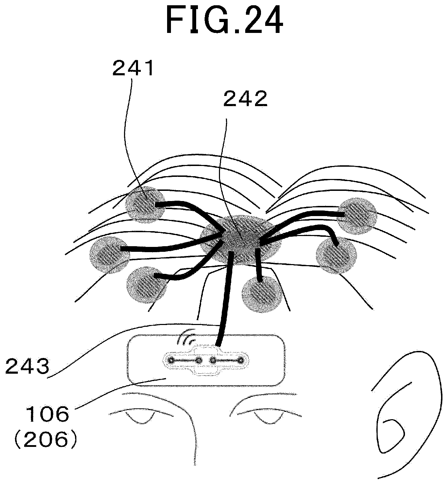

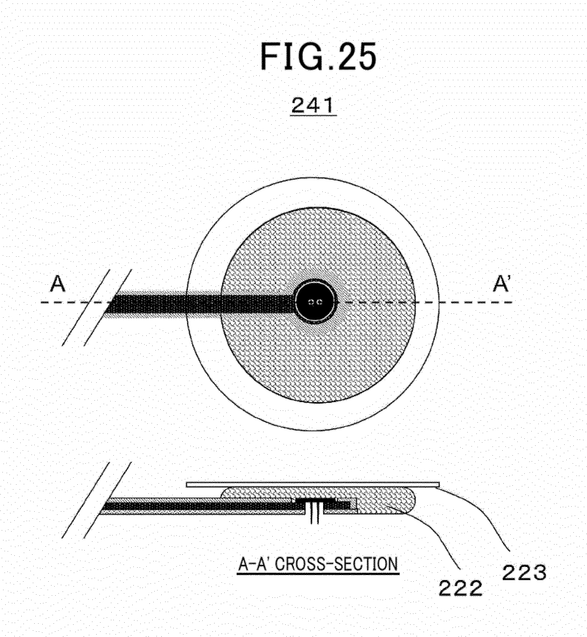

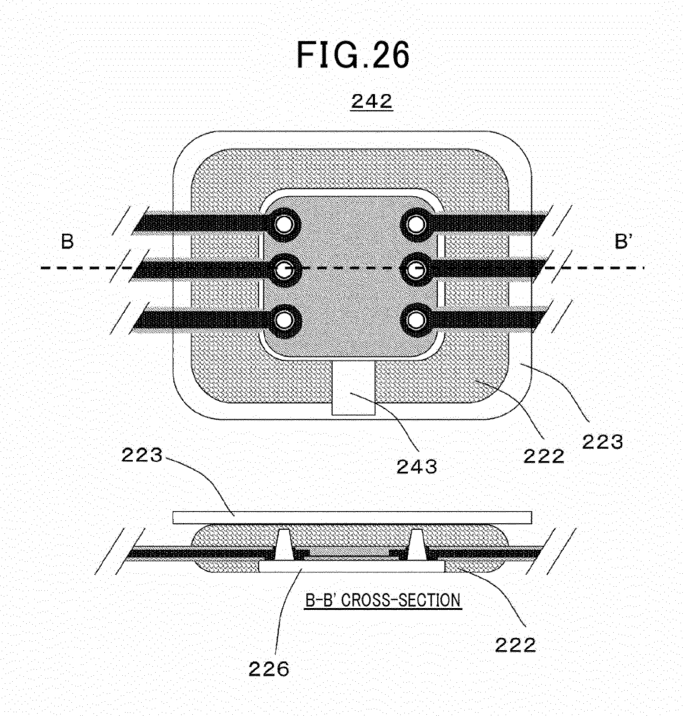

The first to tenth embodiments illustrate covering the whole of the electrode sheet 106 and the sensor module 105, whereas by using the photocurable resin of the tenth embodiment, that is, using the sheet 221 obtained by applying the photocurable resin 222 to the porous film or PET film 223, fixation 241 of the needle-shaped member and fixation 242 of the sensor module 105 or the terminal 226 can, as illustrated in Figs. 24 to 26, separately be achieved in the manner of the tenth embodiment.

By this fixation, it is possible to, separately from the electrode sheet 106 or 206 attached to the forehead, lay separate wiring 243 on a part to be measured, which is located on the periphery of the top of the head and where hair is dense, and to fix needle-shaped members to the tips of the wiring in, for example, a satellite shape. By separating the needle-shaped members from the electrode sheet 106 or 206 attached to the forehead, only the part of the needle-shaped members fixed in the shape of, for example, a satellite can singly be used. This single use enables wearing of 19 electrodes in accordance with the "10-20 system" recommended by the International Congress of Electroencephalography and Clinical Neurophysiology. The application of the eleventh embodiment is not limited to the tenth embodiment, but the eleventh embodiment can be applied for fixation of a biological electrode device. Particularly, the eleventh embodiment is effective when the needle-shaped member is used as an electrode.

### [Twelfth embodiment]

The tenth or eleventh embodiment can also be used as an electrode sheet for electrical therapy while worn on the forehead, the head, the top of the head, the neck, the body, a hand, a leg, or the like.

Embodiments of the present invention have been described in the foregoing. However, the present invention is not limited to the embodiments described above and various alterations are possible within the range not departing from the spirit of the present invention.

Aspects that can correspond to the content of the present invention will be described below. However, the present invention is not limited to these aspects.

### (Aspect 1)

An electrode device for a living body, the electrode device including:
an electrode sheet including
   at least a pair of electrodes that receive a bioelectrical signal,
   a wiring member that transmits the bioelectrical signal received,
   a sensor module that externally outputs a signal relating to the bioelectrical signal, and
   a connector that connects the bioelectrical signal to the sensor module; and
an adhesive sheet that can be attached to a living body while covering the electrode sheet.

### (Aspect 2)

The electrode device according to aspect 1, wherein
the electrode sheet includes a sheet member that supports the electrodes, the wiring member, the sensor module, and the connector.

### (Aspect 3)

The electrode device according to aspect 1 or 2, wherein
the electrodes are needle-shaped members that are brought into contact with the living body and receive the bioelectrical signal.

### (Aspect 4)

The electrode device according to aspect 1 or 2, wherein
the electrode sheet includes at least a pair of conductive gels that is brought into contact with the living body and receives the bioelectrical signal, and
the electrodes are conductive members that receive the bioelectrical signal from the conductive gels.

### (Aspect 5)

The electrode device according to any one of aspects 1 to 4, wherein
the sensor module wirelessly transmits the signal relating to the bioelectrical signal to the exterior.

### (Aspect 6)

The electrode device according to any one of aspects 1 to 4, wherein
the sensor module includes a conductive wire that outputs the signal relating to the bioelectrical signal to the exterior, and
the adhesive sheet can fix a part of the conductive wire to the living body.

### (Aspect 7)

The electrode device according to any one of aspects 1 to 6, wherein
a plurality of pairs of the electrodes are provided, and receive the bioelectrical signal at a plurality of locations.

### (Aspect 8)

The electrode device according to any one of aspects 1 to 7, wherein
a plurality of pairs of the electrodes are provided, and include an electrode that receives the bioelectrical signal and an electrode for electrical therapy that passes an electric current through the living body.

### (Aspect 9)

The electrode device according to any one of aspects 1 to 8, wherein
the wiring member has a meandering pattern.

### (Aspect 10)

The electrode device according to any one of aspects 2 to 9, wherein
the wiring member has a meandering pattern, and
the sheet member includes a slit disposed at any location of the meandering pattern.

### (Aspect 11)

The electrode device according to any one of aspects 2 to 10, wherein
the electrodes are, by fit tolerance of "transition fit" or "tight fit", fixed into places formed by covering with the wiring member a wall surface of holes made in the sheet member.

### (Aspect 12)

The electrode device according to any one of aspects 2 to 11, wherein the connector is, by fit tolerance of "transition fit" or "tight fit", fixed at a location formed by covering with the wiring member a wall surface of a hole made in the sheet member.

### (Aspect 13)

The electrode device according to any one of aspects 1 to 10, wherein
each of the electrodes is fixed so as to be held between the wiring member and the adhesive sheet and have a projection thereof exposed from a hole made in the wiring member.

### (Aspect 14)

The electrode device according to any one of aspects 1 to 10 and 13, wherein
the connector is inserted into and fixed to a hole made in the wiring member.

### (Aspect 15)

The electrode device according to any one of aspects 1 to 14, wherein
the adhesive sheet is porous.

### (Aspect 16)

The electrode device according to any one of aspects 1 to 15, wherein
the adhesive sheet has printing performed thereon with a conductive ink.

### (Aspect 17)

The electrode device according to any one of aspects 1 to 16, wherein
the adhesive sheet has a metal foil attached thereto.

### (Aspect 18)

An electrode device for a living body, the electrode device including:
an electrode sheet including
   at least a pair of electrodes that receive a bioelectrical signal,
   a wiring member that transmits the bioelectrical signal received,
   a sensor module that externally outputs a signal relating to the bioelectrical signal, and
   a connector that connects the bioelectrical signal to the sensor module; and
a photocurable resin that can be attached to a living body while covering the electrode sheet.

### (Aspect 19)

The electrode device according to aspect 18, wherein
the photocurable resin has been applied to a PET film.

### (Aspect 20)

The electrode device according to aspect 18 or 19, wherein
the photocurable resin can separately attach the electrodes and the sensor module to the living body.

### (Aspect 21)

The electrode device according to any one of aspects 18 to 20, wherein
a part or all of the electrodes are used as electrodes for electrotherapy.

### (Aspect 22)

The electrode device according to any one of aspects 1 to 21, wherein
the sensor module is disposed on a living-body-side surface of the electrode sheet.

### [Reference Signs List]

- 101: Needle-shaped member
- 101a: Support
- 101b: Upper flat surface
- 101c: Lower flat surface
- 102: Wiring member
- 103: Connector
- 104: Sheet member
- 104a: Silicone sheet
- 104b: Adhesive layer
- 104c: PET film
- 105: Sensor module
- 106: Electrode sheet
- 107: Adhesive sheet
- 107a: Separation film
- 108: Insulating film
- 111: Release film
- 112a: Insulating film
- 112b: Insulating film
- 114: Range into which needle-shaped member or conductive member is inserted
- 115: Range into which connector is inserted
- 201: Conductive member
- 206: Electrode sheet
- 208: Conductive gel
- 211: Hole
- 221: Sheet
- 222: Photocurable resin
- 223: Porous film or PET film
- 226: Terminal
- 231: Light source
- 232: Hair
- 241: Fixation of needle-shaped member
- 242: Fixation of sensor module or terminal
- 243: Wiring
- 301: Forehead
- 302: Gap
- 401: Hole diameter
- 402: Shaft diameter
- 515: Needle hole
- 901: Through hole
- 1001: Meandering pattern
- 1002: Slit
- 1101: Elastomer material
- 1102: Primer or adhesive agent
- 1201: Part of sensor module
- 1202: Mesh pattern
- 1210: Entire surface
- 1211: Shape covering only part of electrode sheet
- 1212: Shape covering only part of wiring member

## Claims

1. An electrode device for a living body, the electrode device comprising:
an electrode sheet including
at least a pair of electrodes that receive a bioelectrical signal,
a wiring member that transmits the bioelectrical signal received,
a sensor module that externally outputs a signal relating to the bioelectrical signal, and
a connector that connects the bioelectrical signal to the sensor module; and
an adhesive sheet that can be attached to a living body while covering the electrode sheet.

2. The electrode device according to claim 1, wherein
the electrode sheet includes a sheet member that supports the electrodes, the wiring member, the sensor module, and the connector.

3. The electrode device according to claim 1, wherein
the electrodes are needle-shaped members that are brought into contact with the living body and receive the bioelectrical signal.

4. The electrode device according to claim 1, wherein
the electrode sheet includes at least a pair of conductive gels that are brought into contact with the living body and receive the bioelectrical signal, and
the electrodes are conductive members that receive the bioelectrical signal from the conductive gels.

5. The electrode device according to claim 1, wherein
the sensor module wirelessly externally transmits the signal relating to the bioelectrical signal .

6. The electrode device according to claim 1, wherein
the sensor module includes a conductive wire that externally outputs the signal relating to the bioelectrical signal, and
the adhesive sheet fixes fix a part of the conductive wire to the living body.

7. The electrode device according to claim 1, wherein
a plurality of pairs of the electrodes are disposed, and receive the bioelectrical signal at a plurality of locations.

8. The electrode device according to claim 1, wherein
a plurality of pairs of the electrodes are disposed, and include an electrode that receives the bioelectrical signal and an electrode for electrotherapy that passes an electric current through the living body.

9. The electrode device according to claim 1, wherein
the wiring member has a meandering pattern.

10. The electrode device according to claim 2, wherein
the wiring member has a meandering pattern, and
the sheet member includes a slit disposed at any location of the meandering pattern.

11. The electrode device according to claim 2, wherein
the electrodes are, by fit tolerance of "transition fit" or "tight fit", fixed into places formed by covering with the wiring member a wall surface of holes made in the sheet member.

12. The electrode device according to claim 2, wherein
the connector is, by fit tolerance of "transition fit" or "tight fit", fixed at locations formed by covering with the wiring member a wall surface of a hole made in the sheet member.

13. The electrode device according to claim 1, wherein
each of the electrodes is fixed so as to be held between the wiring member and the adhesive sheet and have a projection thereof exposed from a hole in the wiring member.

14. The electrode device according to claim 1, wherein
the connector is inserted into and fixed to a hole in the wiring member.

15. The electrode device according to claim 1, wherein
the adhesive sheet is porous.

16. The electrode device according to claim 1, wherein
the adhesive sheet has printing performed thereon with a conductive ink.

17. The electrode device according to claim 1, wherein
the adhesive sheet has a metal foil attached thereto.

18. An electrode device for a living body, the electrode device comprising:
an electrode sheet including
at least a pair of electrodes that receive a bioelectrical signal,
a wiring member that transmits the bioelectrical signal received,
a sensor module that outputs a signal relating to the bioelectrical signal to an exterior, and
a connector that connects the bioelectrical signal to the sensor module; and
a photocurable resin that can be attached to a living body while covering the electrode sheet.

19. The electrode device according to claim 18, wherein
the photocurable resin is applied to a PET film.

20. The electrode device according to claim 18, wherein
the photocurable resin can separately attach the electrodes and the sensor module to the living body.

21. The electrode device according to claim 18, wherein
a part or all of the electrodes are used as electrodes for electrotherapy.

22. The electrode device according to claim 1 or 18, wherein
the sensor module is disposed on a living-body-side surface of the electrode sheet.
